# NEUE EUROPÄISCHE PATENTSCHRIFT

(11) **EP 2 054 369 B2**
(45) Veröffentlichungstag und Bekanntmachung des Hinweises auf die Entscheidung über den Einspruch: **24.01.2018**
(45) Hinweis auf die Patenterteilung: 17.12.2014
(21) Anmeldenummer: 07803107.7
(22) Anmeldetag: 31.08.2007
(51) Int. Cl.: D21H 19/20, C07C 67/20, C08F 20/10, C09D 4/00, D21H 17/37

(54) **VERFAHREN UND VORRICHTUNG ZUR HERSTELLUNG VON METHACRYLSÄUREALKYLESTERN**
PROCESS AND APPARATUS FOR PREPARING ALKYL ESTERS OF METHACRYLIC ACID
PROCEDE ET DISPOSITIF POUR LA FABRICATION D'ESTERS ALKYLIQUES DE L'ACIDE METHACRYLIQUE

(30) Priorität: 08.12.2006 DE 102006058251
(43) Veröffentlichungstag der Anmeldung: 06.05.2009
(73) Patentinhaber: Evonik Röhm GmbH, 64293 Darmstadt (DE)
(72) Erfinder: GROPP, Udo, 83093 Bad Endorf (DE); WEBER, Robert, 64665 Alsbach-hähnlein (DE); SCHÄFER, Thomas, 64572 Büttelborn (DE); PERL, Andreas, 67240 Bobenheim-Roxheim (DE); SING, Rudolf, 67551 Worms (DE); MERTZ, Thomas, 64625 Bensheim (DE)
(86) Internationale Anmeldenummer: PCT/EP2007/059107
(87) Internationale Veröffentlichungsnummer: WO 2008/068063

(56) Entgegenhaltungen:
- EP-A- 0 686 623
- EP-A1- 1 359 137
- WO-A-02/14388
- DE-B- 1 206 429
- US-A- 2 786 739
- US-A- 3 006 950
- US-A- 3 210 212
- PERP Report Methyl Methacrylate 99/00-2, September 2001, S. 4-10, 12-17

## Beschreibung

Der Gegenstand der vorliegenden Erfindung betrifft ein Verfahren zur Herstellung von Methacrylsäurealkylestern, die im Rahmen einer Vielzahl chemischer Syntheseverfahren eingesetzt werden können, die zu unterschiedlichsten Weiterverarbeitungsprodukten führen können, sowie eine Vorrichtung zur Durchführung dieses Verfahrens.

Zur Herstellung von Methacrylsäurealkylestern existieren bereits seit langer Zeit bekannte und in weiter Verbreitung durchgeführte Verfahren US 3 006 950. Methacrylsäurealkylester werden beispielsweise im Rahmen der Herstellung oligomerer oder polymerer Verbindungen eingesetzt, wobei die entsprechend erhaltenen oligomeren oder polymeren Produkte in einer Vielzahl von Gegenständen, insbesondere auch in einer Vielzahl von Bedarfsgegenständen des täglichen Lebens zu finden sind.

Aufgrund der weiten Verbreitung unterschiedlichster Herstellungsverfahren für Methacrylsäurealkylester und aufgrund der großen benötigten Mengen solcher Verbindungen werden die entsprechenden Verfahren üblicherweise derart dimensioniert, dass die erhaltenen Produktmengen hoch sind. Aus diesem Grund steht die Optimierung bekannter Verfahren bei der Weiterentwicklung der unterschiedlichsten Herstellungsmethoden im Vordergrund. Bereits eine geringfügige Verbesserung im Rahmen eines bekannten Verfahrens kann aufgrund der großen Mengen an hergestelltem Produkt zu erheblichen Vorteilen, beispielsweise zur Verringerung des Energieverbrauchs beim Herstellungsverfahren oder zur Schonung einer im Rahmen des Herstellungsverfahrens benötigten Ressource beitragen.

Die vorliegende Erfindung nimmt sich nun der Problematik an, dass im Rahmen des hier beschriebenen Verfahrens zur Herstellung von Methacrylsäurealkylestern an vielen Stellen Wasser zum Einsatz kommt, wobei dieses Wasser üblicherweise nach dem Durchlaufen des jeweiligen Nutzungszweckes einer Entsorgung zugeführt werden muss. Die vorliegende Erfindung hat es sich daher zur Aufgabe gemacht, den Einsatz von Wasser als Rohstoff in einem Verfahren zur Herstellung von Methacrylsäurealkylestern dahingehend zu optimieren, dass der Wasserverbrauch möglichst minimiert wird und im Rahmen des Verfahrens eingesetztes Wasser möglichst an verschiedenen Stellen innerhalb des Verfahrens im Rahmen verschiedener Verfahrenselemente eingesetzt werden kann.

Darüber hinaus hat sich die vorliegende Erfindung zum Ziel gesetzt, im Rahmen eines verbesserten Wasserkreislaufs die Produktqualität zu erhöhen.

### Zusammenfassung der Erfindung

Die Erfindung betrifft ein Verfahren zur Herstellung von Methacrylsäurealkylestern als Reaktionsprodukt bei dem
a. in einem oder mehreren Reaktionsräume ein Methacrylsäureamid, Wasser, Schwefelsäure und mindestens ein Alkanol enthaltendes Reaktionsgemisch in einer Veresterungsreaktion unterzogen wird,
b. das rohe Reaktionsprodukt in mindestens einer Rektifikationskolonne einer Trennoperation unterzogen wird,
c. das einer Trennoperation unterworfene Reaktionsprodukt in einem oder mehreren Wärmetauschern kondensiert wird,
d. das Kondensat in mindestens einer Scheidevorrichtung in eine organische und eine wässrige Phase getrennt wird,
e. die organische Phase mit Wasser unter Erhalt einer gewaschenen organischen Phase und Waschwasser gewaschen wird und
f. die abgetrennte wässrige Phase zusammen mit dem Waschwasser wieder in mindestens einen Reaktionsraum zurückgeführt wird.

Als Reaktionsraum kann beispielsweise ein Kessel oder eine Kaskade von 2, 3, 4 oder mehr Kesseln eingesetzt werden. Im Rahmen einer weiteren Ausführungsform der Erfindung wird dem Reaktionsraum oder dem ersten von zwei oder mehr Reaktionsräumen zumindest anteilig Wasser zugeführt, das ein Alkanol und Schwefelsäure enthält.

Die Temperatur im Reaktionsraum kann beispielsweise auf etwa 90 bis etwa 120 °C eingestellt werden und dem Reaktionsgemisch kann aus einem oder mehreren Reaktionsräumen dampfförmiges Reaktionsprodukt entzogen werden, das dann beispielsweise über eine Rektifikationskolonne einer Trennoperation unterzogen, in einem oder mehreren Wärmetauschern kondensiert und als Kondensat anschließend mit Wasser gewaschen wird, wobei das so erhaltene Waschwasser vom Reaktionsprodukt durch Phasentrennung abgetrennt und wieder in mindestens einen Reaktionsraum zurückgeführt wird.

Ein erfindungsgemäßes Verfahren kann beispielsweise die folgenden Schritte umfassen:
a. mindestens einem Reaktionsraum wird ein Gemisch enthaltend Wasser, Ammoniumsalze, Reaktionsprodukt, Feststoffe und Schwefelsäure (verbrauchte Säure) entzogen,
b. das Gemisch wird mindestens einem Flotationsbehälter zugeführt wird in dem es durch Flotation weitgehend von Feststoffen befreit wird,
c. der Flotationsbehälter wird derart mit Dampf beaufschlagt, dass das Reaktionsprodukt aus dem Gemisch in die Dampfphase überführt wird,
d. die mindestens Wasser und Reaktionsprodukt enthaltende Dampfphase wird in mindestens einem Wärmetauscher so gekühlt, dass ein Wasser und Reaktionsprodukt enthaltendes Nachkondensat entsteht und
e. das Nachkondensat wird in mindestens einen Reaktionsraum zurückgeführt.

Es hat sich in einigen Fällen als vorteilhaft herausgestellt, wenn die Innenseite mindestens eines Wärmetauschers mit Waschwasser aus der Wäsche des Kondensats aus der Rektifikationskolonne des Vorreinigungsschritts besprüht wird und dabei beispielsweise im Wärmetauscher ein Gemisch aus Nachkondensat und Waschwasser entsteht, das in mindestens einen Reaktionsraum zurückgeführt werden kann.

Oft führt es zu guten Ergebnissen, wenn das Reaktionsprodukt mindestens einer Vorreinigung und mindestens einer Hauptreinigung unterzogen wird, wobei das Reaktionsprodukt bei der Vorreinigung destillativ überwiegend von niedrigsiedenden Verunreinigungen befreit wird, die einen niedrigeren Siedepunkt als der hergestellte Methacrylsäurealkylester aufweisen sowie von deren azeoptropen Gemischen mit Wasser und bei der Hauptreinigung überwiegend von höhersiedenden Verunreinigungen befreit wird, die einen höheren Siedepunkt als der hergestellte Methacrylsäurealkylester aufweisen.

Die niedrigsiedenden Verunreinigungen können dann beispielsweise einer Kondensation in einem oder mehreren Wärmetauschern unterzogen werden, wobei das Kondensat einer Phasentrennung unterzogen werden kann und die wässrige Phase des Kondensats beispielsweise in mindestens einen Reaktionsraum zurückgeführt werden kann.

Erfindungsgemäß hat es sich ebenfalls in einigen Fällen als vorteilhaft herausgestellt, wenn mindestens ein Reaktionsraum mit Methacrylsäureamid und konzentrierter Schwefelsäure beschickt wird.

Erfindungsgemäß kann beispielsweise eine Kaskade von 2, 3 oder 4 Kesseln als Reaktionsräume eingesetzt werden.

Im Rahmen einer weiteren Ausführungsform der Erfindung stammt das im Reaktionsgemisch vorliegenden Wasser zu mindestens 60 Gew.-% aus einem oder mehreren der nachstehenden Verfahrenselemente:
a. Wäsche des rohen Reaktionsprodukts nach einer ersten Trennoperation mit Wasser und anschließendes Abtrennen der wässrigen Phase durch Phasentrennung,
b. Austreiben von Reaktionsprodukt aus der verbrauchten Säure mit Wasserdampf und anschließende Kondensation des so erhaltenen Gemischs aus Reaktionsprodukt und Wasserdampf
c. destillatives Abtrennen niedrigsiedender Verunreinigungen und deren Azeotrope mit Wasser aus dem rohen Reaktionsprodukt, Kondensieren der abgetrennten Verunreinigungen, versetzen des Kondensats mit Wasser und abtrennen der wässrigen Phase durch Phasentrennung.

Ein erfindungsgemäßes Verfahren kann darüber hinaus ein oder mehrere der folgenden Verfahrenselemente umfassen:
a. Herstellung von Acetoncyanhydrin aus Aceton und Blausäure;
b. Reinigung des Acetoncyanhydrins in einer Rektifikationskolonne;
c. Umsetzung des Acetoncyanhydrins zu Methacrylsäureamid.

Ebenfalls Gegenstand der Erfindung ist eine Vorrichtung zur Herstellung von Methacrylsäurealkylester mindestens umfassend
a. einen oder mehrere Reaktionsräume in denen ein Methacrylsäureamid, Wasser Schwefelsäure und ein Alkanol enthaltendes Reaktionsgemisch einer Veresterungsreaktion unterzogen werden kann,
b. mindestens eine Rektifikationskolonne, in der das Reaktionsprodukt einer Trennoperation unterzogen werden kann,
c. einen oder mehrere Wärmetauscher, in denen das einer Trennoperation unterworfene Reaktionsprodukt kondensiert werden kann,
d. mindestens eine Scheidevorrichtung, in der das Kondensat in eine organische und eine wäßrige Phase getrennt werden kann,
e. mindestens eine Waschkolonne, in der die organische Phase mit Wasser gewaschen werden kann, und
f. mindestens eine fluidleitende Verbindung zwischen Scheidevorrichtung und mindestens einem Reaktionsraum, durch welche die abgetrennte wässrige Phase wieder in mindestens einen Reaktionsraum zurückgeführt werden kann.

Eine erfindungsgemäße Vorrichtung kann weiterhin mindestens umfassen:
a. mindestens eine Destillationskolonne, in welcher das rohe Reaktionsprodukt von Verunreinigungen mit einem niedrigeren Siedepunkt als der Methacrylsäurealkylester sowie von deren Azeotropen mit Wasser befreit werden kann;
b. mindestens einen Wärmetauscher, in dem die dampfförmigen abgetrennten Verunreinigungen sowie deren azeotrope mit Wasser unter Bildung eines Kondensats kondensiert werden können,
c. mindestens eine Zuleitung für Wasser, in dem das Kondensat mit Wasser versetzt wird;
d. mindestens eine Scheidevorrichtung, in der eine wässrige Phase des Kondensats von einer organischen Phase getrennt werden kann und
e. mindestens eine fluidleitende Verbindung zwischen der wässrigen Phase und mindestens einem Reaktionsraum;
f. mindestens einen Behälter (Flotationsbehälter), in dem verbrauchte Säure aus der Veresterungsreaktion zumindest teilweise von Feststoffen befreit werden kann;
g. mindestens eine fluidleitende Verbindung zwischen Flotationsbehälter und einem Reaktionsraum;
h. mindestens einen Wärmetauscher zur Kondensation von aus dem Flotationsbehälter entweichenden dampfförmigen Stoffen,
i. mindestens eine fluidleitende Verbindung zwischen Scheidevorrichtung und Wärmetauscher, die eine Zufuhr der in der Scheidevorrichtung erhaltenen wässrigen Phase in das Innere des Wärmetauschers ermöglicht;
j. mindestens eine fluidleitende Verbindung zwischen Wärmetauscher und mindestens einem Reaktionsraum.

### Detaillierte Beschreibung der Erfindung

Unter einem "Reaktionsraum" wird im Rahmen der vorliegenden Erfindung ein Raum verstanden, innerhalb dessen eine erfindungsgemäße Veresterungsreaktion stattfinden kann. Ein Reaktionsraum kann beispielsweise ein Kessel, ein Kolben, ein Schlaufenreaktor oder dergleichen sein. Es ist dabei unerheblich, ob die Veresterungsreaktion im Reaktionsraum kontinuierlich oder batchweise betrieben wird.

Unter einer Veresterungsreaktion wird im Rahmen des vorliegenden Textes eine Reaktion verstanden, deren Reaktionsprodukt ein Methacrylsäurealkylester ist. Vorzugsweise wird unter einer Veresterungsreaktion im Sinne des vorliegenden Textes eine Reaktion zwischen Methacrylsäure und einem linearen oder verzweigten C₁₋₂₂-Monoalkanol, vorzugsweise einem Alkanol mit 1, 2, 3 oder 4 C-Atomen im Alkylrest und einer OH-Gruppe verstanden.

Unter einem "rohen Reaktionsprodukt" wird im Rahmen der vorliegenden Erfindung ein Stoffgemisch verstanden, wie es im Rahmen der Reaktionsführung dem Reaktionsraum entzogen wird. Im Rahmen einer bevorzugten Ausführungsform der vorliegenden Erfindung wird unter einem "rohen Reaktionsprodukt" dasjenige Stoffgemisch verstanden, das einem oder mehreren im Rahmen der Reaktion als Reaktionsraum eingesetzten Kesseln vor jeglicher Weiterverarbeitung oder Reinigung entzogen werden kann.

Unter einer "Rektifikationskolonne" wird im Rahmen des vorliegenden Textes eine Kolonne mit mehreren Böden oder entsprechenden zur Stofftrennung geeigneten Einbauten oder Füllungen verstanden, in der das rohe Reaktionsprodukt einer Trennoperation unterzogen wird. Als Trennoperation wird dabei eine Operation verstanden, bei der ein dem Reaktionsraum entnommenes, rohes Reaktionsprodukt zusammen mit einem als Träger geeigneten Wasserdampf über einen oder mehrere Kolonnenböden soweit aufgereinigt wird, dass im Wesentlichen Wasser, Methacrylsäurealkylester und Alkanol von Verbindungen mit einem höheren Siedepunkt oder von Verbindungen, die im Rahmen der in der Kolonne herrschenden Bedingungen nicht in der Dampfphase aus der Kolonne ausgetragen werden, erzielt wird.

Unter einer "Dampfphase" wird im Rahmen der vorliegenden Erfindung ein gasförmiger Stoff oder ein Gemisch aus zwei oder mehr gasförmigen Stoffen verstanden, das ggf. noch einen Anteil an Flüssigphase enthalten kann.

Unter einer "Waschkolonne" wird im Rahmen der vorliegenden Erfindung eine Vorrichtung verstanden, in der das rohe Reaktionsprodukt nach der Trennoperation mit Wasser gewaschen werden kann. Beispielsweise handelt es sich bei einer geeigneten Waschkolonne um eine Vorrichtung, in der das rohe Reaktionsprodukt nach der Trennoperation im Gegenstrom mit Wasser in Kontakt gebracht werden kann. Bei einer derartigen Operation kann beispielsweise gebildeter Methacrylsäurealkylester nach einer Phasentrennung als organische Phase weiterverarbeitet werden.

Die Erfindung betrifft insbesondere die Hydrolyse von Methacrylsäureamid zu Methacrylsäure und deren gleichzeitiger Veresterung zu Methacrylsäureester. Diese Reaktion kann in einem oder mehreren Reaktionsräumen stattfinden, die im Wesentlichen der Reaktion angemessen beliebig gewählt werden können. Insbesondere haben sich beheizte, beispielsweise durch Dampf beheizte Kessel als geeignet herausgestellt. Es hat sich in vielen Fällen als vorteilhaft erwiesen, wenn die Veresterung in mindestens zwei aufeinander folgenden Kesseln als Reaktionsräume, beispielsweise jedoch auch in drei oder vier oder mehr aufeinander folgenden Kesseln durchgeführt wird. Dabei wird eine Lösung von Methacrylsäureamid in den Kessel bzw. in den ersten Kessel einer zwei oder mehr Kessel umfassenden Kaskade von Kesseln eingeführt.

Es ist erfindungsgemäß häufig bevorzugt, eine entsprechende Veresterungsreaktion mit einer Kaskade von zwei oder mehr Kesseln als Reaktionsräume durchzuführen. Im Folgenden soll daher ausschließlich auf diese Variante Bezug genommen werden.

Im Rahmen des hier beschriebenen Verfahrens kann beispielsweise eine Amidlösung, wie sie aus der im vorliegenden Text beschriebenen Amidierungsreaktion erhältlich ist, in einen ersten Kessel eingespeist werden. Der Kessel wird beispielsweise mit Dampf beheizt. Die zugeführte Amidlösung weist in der Regel eine erhöhte Temperatur auf, beispielsweise eine Temperatur von etwa 100 bis etwa 180 °C, im Wesentlichen entsprechend der Ablauftemperatur der Amidlösung aus der oben vorgestellten Amidierungsreaktion. Den Kesseln wird weiterhin ein Alkanol zugeführt, das zur Veresterung eingesetzt werden kann.

Grundsätzlich eignen sich hier beliebige Alkanole mit 1 bis etwa 22 Kohlenstoffatomen, die linear oder verzweigt, gesättigt oder ungesättigt sein können, wobei Methanol besonders bevorzugt ist. Ebenso können diese Alkanole zusammen mit Methacrylsäureestern eingesetzt werden, was insbesondere bei Umesterungen der Fall ist.

Der Kessel wird weiterhin mit Wasser beschickt, so dass insgesamt eine Wasserkonzentration im Kessel von etwa 13 bis etwa 26 Gew.-%, insbesondere etwa 18 bis etwa 20 Gew.-% herrscht. Es kann dabei erfindungsgemäß bevorzugt sein, wenn dem Reaktionsraum Wasser zugeführt wird, das beispielsweise ein Alkanol oder ein Gemisch aus zwei oder mehr Alkanolen und Schwefelsäure enthält. Als Alkanole eignen sich insbesondere diejenigen Alkanole, die im Rahmen der Veresterungsreaktion im Reaktionsgemisch vorliegen.

Die Menge an Amidlösung und an Alkanol wird derart geregelt, dass ein Gesamtmolverhältnis von Amid zu Alkanol von etwa 1:1,4 bis etwa 1:1,6 herrscht. Das Alkanol kann auf die Kesselkaskade derart verteilt werden, dass im ersten Reaktor das Molverhältnis etwa 1:1,1 bis etwa 1:1,4 beträgt und in den folgenden Reaktionsstufen bezogen auf den Gesamtamidstrom Molverhältnisse von etwa 1:0,05 bis etwa 1:0,3 eingestellt werden. Das in die Veresterung zugeführte Alkanol kann sich aus "Frischalkanol" sowie Alkanol aus Recyclingströmen der Aufarbeitungsstufen und bei Bedarf auch aus Recyclingströmen der Downstreamprozesse des Produktionsverbundes zusammensetzen.

Die Beschickung des ersten Kessels mit Wasser kann grundsätzlich dahingehend erfolgen, dass Wasser aus einer beliebigen Quelle dem Kessel zugeführt wird, sofern dieses Wasser keine Inhaltsstoffe aufweist, die die Veresterungsreaktion oder die nachfolgenden Prozessstufen nachteilig beeinflussen könnten. Beispielsweise kann dem Kessel VE-Wasser oder Brunnenwasser zugeführt werden. Es ist jedoch im Rahmen der vorliegenden Erfindung vorgesehen, dem Kessel als Reaktionsraum anteilig ein Gemisch aus Wasser und organischen Verbindungen zuzuführen, wie sie beispielsweise bei der Reinigung von Methacrylsäure oder Methacrylsäureestern anfallen. Im Rahmen einer weiteren Ausführungsform des hier vorgestellten Verfahrens werden die Kessel zumindest in einem Anteil von mindestens etwa 50 Gew.-%, beispielsweise mindestens etwa 60 oder mindestens etwa 70 oder mindestens etwa 80 oder mindestens etwa 90 Gew.-% mit einem Gemisch aus Wasser und solchen organischen Verbindungen beschickt.

Wenn eine Kaskade von zwei oder mehr Kesseln im Rahmen der Veresterungsreaktion eingesetzt wird, so können die entstandenen dampfförmigen bzw. gasförmigen Stoffe (beide begriffe werden hier synonym verwendet), insbesondere der Methacrylsäureester, grundsätzlich aus jedem Kessel einzeln abgezogen und einer Reinigung zugeführt werden. Es hat sich jedoch in manchen Fällen als vorteilhaft erwiesen, wenn bei einer Kaskade von zwei oder mehr Kesseln die gasförmigen Produkte aus dem ersten Kessel zunächst in den zweiten Reaktionskessel eingespeist werden, ohne dass die gasförmigen Verbindungen aus dem ersten Kessel direkt einer Reinigung zugeführt werden. Diese Vorgehensweise bietet den Vorteil, dass der im ersten Kessel häufig starken Schaumentwicklung nicht durch aufwendige, apparative Entschäumung begegnet werden muss. Im Falle einer Kaskadierung der gasförmigen Stoffe aus dem ersten Kessel in den zweiten Kessel tritt der im ersten Kessel gebildete und ggf. mitgerissene Schaum einfach in den Reaktionsraum des zweiten Kessels mit ein. Da dort in der Regel die Schaumbildung deutlich geringer ist, muss so nicht apparativ entschäumt werden.

Der nach einem ersten Kessel angeordnete zweite Kessel nimmt nun einerseits den Überlauf des ersten Kessels auf, andererseits wird er mit den gasförmigen im ersten Kessel gebildeten oder im ersten Kessel vorhandenen Stoffen gespeist. Der zweite Kessel und die evt. folgenden werden ebenfalls mit Methanol beschickt. Hierbei ist es bevorzugt, dass die Methanolmenge von Kessel zu Kessel um mindestens 10 %, jeweils auf den vorhergehenden Kessel bezogen, abnimmt. Die Wasserkonzentration im zweiten Kessel sowie in den weiteren Kesseln kann sich von der des ersten Kessels unterscheiden, oftmals sind die Konzentrationsunterschiede jedoch gering.

Die Regelung der Wasserkonzentration ist beispielsweise über die Zufuhr an bereits im Verfahenskreislauf befindlichem Wasser und über den Austrag von Wasser aus den Reaktionsräumen möglich, In diesem Zusammenhang ist es erfindungsgemäße von Vorteil, dass das rohe Reaktionsprodukt mit Wasserdampf als Träger aus den Reaktionsräumen ausgetrieben werden kann und anschließend in einer Rektifikationskolonne einer Trennoperation unterzogen werden kann, wobei ein Gemisch aus Wasser und organischen Verbindungen, insbesondere Methacrylsäurealkylester, abgetrennt wird, aus dem wiederum das Wasser wie beschrieben zurück gewonnen werden kann.

Die im zweiten Kessel entstehenden Brüden werden aus dem Kessel abgeführt und in den Sumpf einer Destillationskolonne bzw. einer Rektifikationskolonne eingeleitet.

Wenn die Veresterung mit einer Kaskade von drei oder mehr Kesseln durchgeführt wird, so wird jeweils der Überlauf des zweiten Kessels in einen dritten Kessel überführt sowie der Überlauf des dritten Kessels ggf. in einen vierten Kessel überführt. Die weiteren Kessel werden ebenfalls dampfbeheizt. Vorzugsweise wird die Temperatur in den Kesseln 3 und ggf. 4 auf etwa 120 °C bis etwa 140 °C eingestellt.

Die aus den Kesseln entweichenden Brüden können in eine Destillationskolonne bzw. in eine Rektifikationskolonne eingeleitet werden, wobei die Einleitung vorzugsweise im Unterbereich der Kolonne erfolgt. Die Brüden umfassen ein azeotropes Gemisch aus Träger-Dampf, Methacrylsäureester und Alkanol und weisen je nach eingesetztem Alkanol eine Temperatur von etwa 60 bis etwa 120 °C, beispielsweise etwa 70 bis etwa 90 °C bei Einsatz von Methanol auf. In der Destillationskolonne wird der Methacrylsäureester gasförmig von den bei höheren Temperaturen siedenden Brüdenbestandteilen abgetrennt. Die hochsiedenden Anteile (hauptsächlich Methacrylsäure, Hydroxyisobuttersäureester und Wasser) werden beispielsweise in den ersten Reaktionskessel zurückgeführt. Der gebildete Methacrylsäureester kann am Kolonnenkopf abgezogen und über einen Wärmetauscher oder eine Kaskade von zwei oder mehr Wärmetauschern abgekühlt werden. Es hat sich in einigen Fällen bewährt, wenn die Kühlung des Methacrylsäureesters über mindestens zwei Wärmetauscher erfolgt, wobei ein erster Wärmetauscher mit Wasser die Kondensation und eine Kühlung auf eine Temperatur von etwa 60 bis etwa 30 °C durchführt, während ein zweiter, solegekühlter Wärmetauscher eine Abkühlung auf etwa 5 bis etwa 15 °C vornimmt. Von dem wassergekühlten Kondensat kann ein Teilstrom als Rücklauf auf die Kolonnen zur Konzentrationssteuerung der Kolonne gegeben. Es ist jedoch ebenso möglich, den gebildeten Methacrylsäureester über eine Kaskade von mehr als zwei Wärmetauschern zu kühlen. Hierbei ist es beispielsweise möglich, zunächst eine Kühlung über zwei hintereinander geschaltete, wassergekühlte Wärmetauscher vorzunehmen und anschließend über einen entsprechenden solegekühlten Wärmetauscher eine weitere Abkühlung zu erzielen.

So kann beispielsweise im Rahmen des hier vorgestellten Verfahrens der gebildete Methacrylsäureester in gasförmigem Zustand über einen ersten Wärmetauscher mit Wasserkühlung gekühlt werden. Sowohl kondensierte als auch nicht kondensierte Stoffe werden anschließend in einen zweiten Wärmetauscher weitergeleitet, wo eine weitere Kondensation über Wasserkühlung stattfindet. An dieser Stelle können nun beispielsweise gasförmige Stoffe in einen separaten, mit Sole gekühlten Wärmetauscher überführt werden. Das Kondensat in diesem solegekühlten Wärmetauscher wird anschließend in den Destillatstrom gegeben, während die verbleibenden gasförmigen Stoffe weiter verwertet werden können oder einer Entsorgung zugeführt werden. Das Methacrylsäureester-Kondensat aus dem zweiten wassergekühlten Wärmetauscher wird nun in einem mit Wasser oder mit Sohle gekühlten Wärmetauscher auf eine Temperatur von weniger als 15 °C, vorzugsweise etwa 8 bis etwa 12 °C gekühlt. Dieser Kühlschritt kann dazu führen, dass der gebildete Methacrylsäureester einen deutlich niedrigeren Gehalt an Ameisensäure aufweist, als dies ohne den entsprechenden Kühlungsschritt der Fall wäre. Das abgekühlte Kondensat wird anschließend in einen Phasentrenner überführt. Hier wird die organische Phase (Methacrylsäureester) von der wässrigen Phase getrennt. Die wässrige Phase, die neben Wasser noch einen Gehalt an organischen Verbindungen, insbesondere Alkanol und gegebenenfalls weiteren Verunreinigungen, aus dem Destillationsschritt aufweisen kann, kann grundsätzlich beliebig weiterverwendet werden.

Die abgetrennte organische Phase wird in einen Wäscher eingespeist. Dort wird der Methacrylsäureester mit demineralisiertem Wasser gewaschen. Dieser Verfahrensschritt bietet insbesondere den Vorteil, dass oft Verbindungen, die in nachfolgenden Schritten zu Verfärbungen führen können, weitgehend aus dem Produkt entfernt werden können. Die abgeschiedene wässrige Phase, die ein Gemisch aus Wasser und organischen Verbindungen, insbesondere Alkanol und eventuell weiteren Verunreinigungen, enthält, kann wiederum grundsätzlich beliebig weiterverwendet werden. Es ist jedoch unter ökonomischen Gesichtspunkten vorteilhaft, diese wässrige Phase wieder in den Veresterungsschritt zurück zu führen, indem sie beispielsweise in den ersten Kessel eingespeist wird.

Da Methacrylsäureester eine starke Neigung zur Polymerisation aufweisen, ist es in vielen Fällen vorteilhaft, wenn im Rahmen der Veresterung von Methacrylsäure dafür Sorge getragen wird, dass eine derartige Polymerisation verhindert wird.

In Anlagen zur Herstellung von Methacrylsäure oder Methacrylsäureestern findet Polymerisation oft dann statt, wenn Methacrylsäure oder Methacrylsäureester einerseits eine geringe Strömungsgeschwindigkeit aufweisen, so dass sich lokal Ruhezonen ausbilden können, in denen ein über längere Zeit andauernder Kontakt zwischen Methacrylsäure oder Methacrylsäureester und einem Polymerisationsinitiator einstellen kann, der in Folge dann zur Polymerisation führen kann.

Um ein entsprechendes Polymerisationsverhalten zu vermeiden, kann es vorteilhaft sein, eine Optimierung des Stoffflusses dahingehend durchzuführen, dass einerseits die Strömungsgeschwindigkeit des Methacrylsäureesters oder der Methacrylsäure an möglichst allen Stellen im System so hoch ist, dass die Zahl der Ruhezonen minimiert wird. Darüber hinaus kann es vorteilhaft sein, den Strom an Methacrylsäure bzw. Methacrylsäureester derart mit geeigneten Stabilisatoren zu versetzen, dass eine Polymerisation weitgehend unterdrückt wird.

Zu diesem Zweck können im Rahmen des hier dargestellten Verfahrens grundsätzlich die Stoffströme derart mit Stabilisatoren versetzt werden, dass möglichst wenig Polymerisation im System selbst stattfindet. Hierzu wird insbesondere der Teil der Anlage mit entsprechenden Stabilisatoren versorgt, in dem die Methacrylsäure bzw. der Methacrylsäureester während oder nach der Destillation in hoher Konzentration vorliegt.

So hat es sich beispielsweise als sinnvoll erwiesen, am Kopf der Destillationskolonne dem dort abgezogenen Strom an Methacrylsäureester einen Stabilisator zuzuführen. Weiterhin hat es sich als vorteilhaft erwiesen, diejenigen Anlagenteile mit einer Lösung von Stabilisator in Methacrylsäureester zu spülen, in denen Methacrylsäure oder Methacrylsäureester mit einer Temperatur von mehr als etwa 20 °C, vorzugsweise mit einer Temperatur im Bereich von etwa 20 bis etwa 120 °C zirkuliert. So wird beispielsweise ein Teil des in den Wärmetauschern anfallenden Kondensates zusammen mit einem geeigneten Stabilisator derart in den Kopf der Destillationskolonne zurückgeführt, dass dort der Kolonnenkopf an seiner Innenseite ständig mit stabilisiertem Methacrylsäureester oder stabilisierter Methacrylsäure besprüht wird. Dies geschieht vorzugsweise derart, dass sich im Kolonnenkopf keine Ruhezonen ausbilden können, an denen eine Polymerisation von Methacrylsäure oder Methacrylsäureester zu befürchten ist. Die Wärmetauscher selbst können entsprechend ebenfalls mit einer stabilisierten Lösung von Methacrylsäure oder Methacrylsäureester derart beaufschlagt werden, dass auch hier keine Ruhezonen zur Ausbildung gelangen können.

Es hat sich weiterhin als Vorteil im Rahmen des hier vorgestellten Verfahrens gezeigt, wenn beispielsweise die Kohlenmonoxid enthaltenden Abgase aus vorangehenden Prozessen, insbesondere aus dem Amidierungsschritt, zusammen mit Dampf durch die Veresterungsanlage geleitet werden. Auf diese Weise erfolgt eine nochmalige Vereinigung des Gasgemisches von Verbindungen, die als Feststoff oder als Flüssigkeit abgetrennt werden können. Zum anderen werden diese an einer zentralen Stelle gesammelt und können der weiteren Verwertung oder Entsorgung zugeführt werden.

Das im Rahmen der Veresterung und der anschließenden Vorreinigung erhaltene MMA bzw. der erhaltene Methacrylsäureester oder die erhaltene Methacrylsäure werden anschließend einer weiteren Behandlung zugeführt. Aus der Veresterung resultiert als verbleibender Reststoff verdünnte Schwefelsäure, die ebenfalls einer weiteren Verwertung zugeführt'werden kann.

### Vorreinigung des Esters oder der Säure

Im Rahmen des hier vorgestellten Verfahrens kann der Gegenstand der vorliegenden Erfindung auch im Zusammenhang mit einem Verfahren zur Vorreinigung von Methacrylsäure oder Methacrylsäureester eingesetzt werden, wie dieses in dem nachfolgenden Verfahrenselement beschrieben wird. So wird grundsätzlich rohe Methacrylsäure oder ein roher Methacrylsäureester einer weiteren Reinigung unterzogen, um zu einem möglichst reinen Produkt zu gelangen. Eine derartige ein weiteres Verfahrenselement darstellende Reinigung kann beispielsweise einstufig sein. Es hat sich jedoch in vielen Fällen als vorteilhaft herausgestellt, wenn eine solche Reinigung mindestens zwei Stufen umfasst, wobei in einer ersten Vorreinigung, wie sie hier beschrieben wird, die niedrigsiedenden Bestandteile des Produktes entfernt werden. Hierzu wird roher Methacrylsäureester bzw. rohe Methacrylsäure zunächst in eine Destillationskolonne überführt, in welcher die niedrigsiedenden Bestandteile und Wasser abgetrennt werden können. Hierzu wird der rohe Methacrylsäureester einer Destillationskolonne zugeführt, wobei die Zugabe etwa in der oberen Hälfte der Kolonne durchgeführt wird. Der Kolonnensumpf wird mit Dampf beispielsweise derart beheizt, dass eine Wandtemperatur von etwa 50 bis etwa 120 °C erreicht wird. Die Reinigung wird unter Vakuum durchgeführt. Der Druck innerhalb der Kolonne beträgt im Fall des Esters vorzugsweise etwa 100 bis etwa 600 mbar. Der Druck innerhalb der Kolonne beträgt im Fall der Säure vorzugsweise etwa 40 bis etwa 300 mbar.

Am Kolonnenkopf werden die niedrigsiedenden Bestandteile abgenommen. Insbesondere können dieses beispielsweise Ether, Aceton und Methylformiat sein. Die Brüden werden anschließend über einen oder mehrere Wärmetauscher kondensiert. Dabei hat es sich beispielsweise in einigen Fällen bewährt, zunächst eine Kondensation über zwei in Serie geschaltete, mit Wasser gekühlte Wärmetauscher durchzuführen. Es ist jedoch ebenso möglich, an dieser Stelle auch nur einen Wärmetauscher einzusetzen. Die Wärmetauscher werden vorzugsweise zur Erhöhung der Flussgeschwindigkeit und um die Ausbildung stationärer Phasen zu verhindern, in senkrechtem Zustand betrieben. Dem wassergekühlten Wärmetauscher oder den wassergekühlten Wärmetauschern nachgeschaltet, kann ein solegekühlter Wärmetauscher sein, es ist jedoch auch möglich, eine Kaskade von zwei oder mehr solegekühlten Wärmetauschern nachzuschalten. In der Kaskade von Wärmetauschern werden die Brüden kondensiert, mit Stabilisator versehen und beispielsweise einem Phasentrenner zugeführt. Da die Brüden auch Wasser enthalten können, wird eine evtl. anfallende wässrige Phase entsorgt oder einer weiteren Verwertung zugeführt. Als weitere Verwertung bietet sich beispielsweise die Rückführung in eine Veresterungsreaktion, beispielsweise in eine Veresterungsreaktion wie sie oben beschrieben wurde, an. In diesem Fall wird die wässrige Phase vorzugsweise in den ersten Veresterungskessel rückgeführt.

Die abgetrennte organische Phase wird als Rückfluss in den Kolonnenkopf eingespeist. Ein Teil der organischen Phase kann wiederum zum Besprühen der Wärmetauscherköpfe und des Kolonnenkopfes eingesetzt werden. Da es sich bei der abgetrennten organischen Phase um eine Phase handelt, die mit Stabilisator versetzt ist, lässt sich so wirksam einerseits die Ausbildung von Ruhezonen verhindern. Andererseits bewirkt die Gegenwart des Stabilisators eine weitere Unterbindung der Polymerisationsneigung der abgetrennten Brüden.

Der aus den Wärmetauschern erhaltene Kondensatstrom wird darüber hinaus vorzugsweise derart mit demineralisiertem Wasser versetzt, dass im Phasentrenner eine ausreichende Abscheidwirkung erzielt werden kann.

Die nach der Kondensation in der Wärmetauscherkaskade verbleibenden, gasförmigen Verbindungen können, vorzugsweise mittel Dampfstrahler als Unterdruckerzeuger, nochmals über einen oder mehrere weitere Wärmetauscher einer Kondensation unterzogen werden. Es hat sich dabei unter ökonomischen Gesichtspunkten als vorteilhaft herausgestellt, wenn im Rahmen einer solchen Nachkondensation nicht nur die gasförmigen Stoffe aus der Vorreinigung kondensiert werden. So ist es beispielsweise möglich, einer derartigen Nachkondensation weitere gasförmige Stoffe zuzuführen, wie sie sich aus der Hauptreinigung von Methacrylsäureestern ergeben. Der Vorteil einer derartigen Verfahrensweise liegt beispielsweise darin, dass so ein Anteil an Methacrylsäureester, der im Rahmen der Hauptreinigungsstufe nicht kondensiert wurde, im Rahmen der Vorreinigung nochmals über den Phasenabscheider in die Reinigungskolonne überführt werden kann. So wird beispielsweise gewährleistet, dass eine Ausbeutemaximierung stattfinden kann, und möglichst geringe Verluste an Methacrylsäureester auftreten. Außerdem kann durch die geeignete Wahl der Auslegung und des Betriebs dieser weiteren Wärmetauscher die Zusammensetzung des diese Wärmetauscher verlassenden Abgases, insbesondere der Gehalt an Leichtsiedern, eingestellt werden.

Aufgrund der Zuführung von Wasser im Rahmen der Vorreinigung des Methacrylsäureesters kann der Wassergehalt in der Veresterung und die Konzentration an niedrigsiedenden Bestandteilen im Roh-Methylmethacrylat insgesamt kontinuierlich ansteigen. Um dies zu vermeiden, kann es vorteilhaft sein, einen Teil des dem System zugeführten Wassers, vorzugsweise kontinuierlich, aus dem System auszuschleusen. Dieses Ausschleusen kann grundsätzlich beispielsweise in einer Größenordnung erfolgen, in der in der Vorreinigung dem System Wasser zugeführt wird. Die im Phasentrenner abgeschiedene wässrige Phase weist üblicherweise einen Gehalt an organischen Inhaltsstoffen auf. Es kann daher vorteilhaft sein, dieses Wasser einer Form der Entsorgung zuzuführen, die diesen Gehalt an organischen Stoffen ausnutzt.

So kann es beispielsweise vorteilhaft sein, wenn ein derart mit organischen Stoffen belastetes Wasser im Rahmen eines Schwefelsäurespaltverfahrens dem Brennraum zugemischt wird. Aufgrund der oxidierbaren Inhaltsstoffe kann so deren Brennwert, zumindest teilweise, noch genutzt werden. Darüber hinaus wird so eine möglicherweise kostspielige Entsorgung des mit organischen Stoffen belasteten Wassers oft vermieden.

### Feinreinigung des Methacrylsäureesters

Zur Feinreinigung des Methacrylsäureesters wird der vorgereinigte Methacrylsäureester einer erneuten Destillation unterzogen. Dabei wird der rohe Methacrylsäureester mit Hilfe einer Destillationskolonne von seinen hochsiedenden Bestandteilen befreit und so ein Reinmethacrylsäureester erhalten. Hierzu wird der rohe Methacrylsäureester in einer dem Fachmann bekannten Weise in die untere Hälfte einer Destillationskolonne eingebracht.

Die Destillationskolonne kann grundsätzlich einer beliebigen, dem Fachmann geeignet erscheinenden Ausführung entsprechen. Es hat sich jedoch für die Reinheit des erhaltenen Produkts in vielen Fällen als vorteilhaft herausgestellt, wenn die Destillationskolonne mit einer oder mehreren Packungen betrieben wird, die den folgenden Vorgaben etwa entspricht:
Zum einen sollen sich in den Kolonnen genauso wie in den anderen mit Methacrylsäureester durchströmten Leitungen möglichst wenig so genannte "Toträume" bilden. Die Toträume führen zu einer vergleichsweise langen Verweilzeit der Methacrylsäureester, die deren Polymerisation begünstigen. Dieses führt wiederum zu kostspieligen Produktionsunterbrechungen und Reinigungen der entsprechenden mit Polymer zugesetzten Teile. Der Bildung von Toträumen kann unter anderem dadurch begegnet werden, dass sowohl durch Auslegung als auch durch eine geeignete Betriebsweise der Kolonnen, diese stets mit einer ausreichenden Menge von Flüssigkeit beladen werden, so dass eine ständige Umspülung der Kolonnen und insbesondere der Kolonneneinbauten wie Packungen erreicht wird. So können die Kolonnen Sprüheinrichtungen aus, die zum Besprühen der Kolonneneinbauten ausgelegt sind auf. Weiterhin können die Kolonneneinbauten untereinander oder mit der Kolonne über unterbrochene Haftnähte verbunden sein. Derartige Haftnähte weisen mindestens etwa 2, vorzugsweise mindestens etwa 5 und besonders bevorzugt mindestens etwa 10 Unterbrechungen auf 1 m Haftnahtlänge auf. Die Länge dieser Unterbrechungen kann so gewählt sein, dass diese mindestens etwa 10, vorzugsweise mindestens etwa 20 und besonders bevorzugt mindestens etwa 50 %, im allgemeinen aber nicht mehr als 95 % der Haftnahtlänge ausmachen. Eine andere bauliche Maßnahme kann darin liegen, dass in den Kolonneninnenbereichen, insbesondere die mit der Methacrylsäureester in Kontakt kommen, weniger als etwa 50 %, vorzugsweise weniger als etwa 25 % und besonders bevorzugt weniger als etwa 10 % aller Flächen, insbesondere von Kolonneneinbauten, horizontal verlaufen. So können beispielsweise die in das Innere der Kolonne mündenden Stutzen konisch bzw. mit schrägen Flächen ausgestaltet sein. Ferner kann eine Maßnahme darin bestehen, die während des Betriebes der Kolonne im Kolonnensumpf befindliche Menge flüssiger von Methacrylsäureestern so gering wie möglich zu halten und anderseits eine Überhitzung dieser Menge trotz moderaten Temperaturen und großen Verdampfungsflächen während des Verdampfen zu vermeiden. Hierbei kann es vorteilhaft sein, dass die Flüssigkeitsmenge im Kolonnesumpf im Bereich von etwa 0,1 bis 15 % und vorzugsweise etwa 1 bis 10 % der Gesamtmenge an Methacrylsäureester in der Kolonne ausmacht. Die in diesem Abschnitt vorgeschlagenen Maßnahmen können auch bei der Destillation von Methacrylsäure Anwendung finden.

Im Rahmen der Reinigung des Methacrylsäureesters werden dessen hochsiedende Bestandteile durch Destillation vom Produkt getrennt. Hierzu wird der Kolonnensumpf mit Dampf beheizt. Die Sumpftemperatur beträgt dabei vorzugsweise etwa 50 bis etwa 80 °C, insbesondere etwa 60 bis etwa 75 °C bei Wandtemperatur von weniger als etwa 120 °C.

Das im Kolonnensumpf anfallende Material wird vorzugsweise kontinuierlich abgeführt und über einen Wärmetauscher oder eine Kaskade von mehreren Wärmetauschern auf eine Temperatur in einem Bereich von etwa 40 bis etwa 80 °C, vorzugsweise etwa 40 bis etwa 60 °C und besonders bevorzugt in einem Bereich von etwa 50 bis 60 °C abgekühlt.

Dieses Material, das überwiegend Methacrylsäureester, Hydroxyisobuttersäureester, Methacrylsäure und Stabilisatorkomponenten enthält, wird anschließend über einen Lagerbehälter, beispielsweise entsorgt oder einer anderweitigen Verwendung zugeführt. Es hat sich in vielen Fällen als vorteilhaft erwiesen, wenn das im Kolonnensumpf gewonnene Material in die Veresterungsreaktion zurückgeführt wird. Beispielsweise wird dabei das Material aus dem Kolonnensumpf in den ersten Kessel rückgeführt. Daraus ergibt sich der Vorteil, dass im Hinblick auf eine möglichst wirtschaftliche Fahrweise und eine möglichst hohe Ausbeute im Kolonnensumpf enthaltene, höhersiedende Verbindungen in die Veresterungsreaktion zurückgeführt werden.

Am Kolonnenkopf wird der destillativ aufgereinigte Methacrylsäureester entnommen und über einen Wärmetauscher oder eine Kaskade von zwei oder mehr Wärmetauschern gekühlt. Dabei kann die Wärme der Brüden durch wassergekühlte Wärmetauscher oder durch solegekühlte Wärmetauscher oder durch eine Kombination aus beidem abgeführt werden. Es hat sich in einigen Fällen bewährt, wenn die Brüden aus der Destillationskolonne in zwei oder mehr parallel geschaltete Wärmetauscher überführt werden, die mittels Wasserkühlung betrieben werden. Die nicht kondensierten Anteile aus den wassergekühlten Wärmetauschern können beispielsweise in einen solegekühlten Wärmetauscher oder eine Kaskade von zwei oder mehr solegekühlten Wärmetauschern eingeleitet werden, die seriell oder parallel angeordnet sein können. Die aus den Wärmetauschern erhältlichen Kondensate werden in einen Sammelbehälter eingeleitet und mittels einer Pumpe über einen weiteren Wärmetauscher oder eine Kaskade von zwei oder mehr weiteren Wärmetauschern einem Pufferbehälter zugeführt. Der Kondensatstrom wird dabei beispielsweise über eine Kaskade von einem oder zwei wassergekühlten Wärmetauschern und einem oder zwei solegekühlten Wärmetauschern auf eine Temperatur in einem Bereich von etwa 0 bis etwa 20 °C, vorzugsweise etwa 0 bis etwa 15 °C und besonders bevorzugt in einem Bereich von etwa 2 bis 10 °C heruntergekühlt.

Dem Kondensatstrom wird ein Teilstrom entnommen, der über den Kolonnenkopf in die Destillationskolonne zurückgeführt wird. Die Einspeisung des Kondensatstromes in den Kolonnenkopf kann dabei grundsätzlich auf beliebige Weise z.B. über Verteiler erfolgen. Es kann jedoch vorteilhaft sein, wenn ein Teil des Kondensatstromes oberhalb des Kolonnenkopfes in die Brüdenleitung eingespeist, beispielsweise eingesprüht, wird. Weiterhin ist es bevorzugt, dass mit dieser Einspeisung Stabilisator in den Kolonnenkopf eingetragen wird.

Ein weiterer Teilstrom des zur Rückführung in die Kolonne vorgesehenen Kondensates kann beispielsweise vor Einbringung in die Brüdenleitung abgezweigt und direkt in den Kolonnenkopf eingebracht werden. Auch hier ist es bevorzugt, dass mit dieser Einspeisung Stabilisator in den Kolonnenkopf eingetragen wird. Die Einbringung in den Kolonnenkopf kann dabei beispielsweise dergestalt geschehen, dass das Innere des Kolonnenkopfes mit dem Kondensat so besprüht wird, dass sich keine Ruhezonen im Kolonnenkopf ausbilden können, an denen eine Polymerisation des Methacrylsäureesters erfolgen kann. Es kann darüber hinaus vorteilhaft sein, wenn einem Kondensatteilstrom, der in die Kolonne zurückgeführt wird, ein Stabilisator zur Verhinderung von Polymerisation zugefügt wird. Dies kann beispielsweise dadurch geschehen, dass dem zum Besprühen des Kolonnenkopfes vorgesehenen Kondensatteilstrom eine entsprechende Menge an Polymerisationsinhibitor als Stabilisator zugefügt wird. Dabei hat es sich in einigen Fällen als vorteilhaft erwiesen, wenn der Kondensatteilstrom nach der Zugabe des Stabilisators, aber vor dem Eintritt in den Kolonnenkopf eine geeignete Mischvorrichtung, vorzugsweise einen Statikmischer durchläuft, um eine möglichst uniforme Verteilung des Stabilisators im Kondensatteilstrom zu erzielen.

Die im Rahmen des Reinigungsverfahrens anfallenden, nicht kondensierbaren gasförmigen Stoffe werden beispielsweise der Entsorgung zugeführt.

Das im Pufferbehälter befindliche Rohprodukt wird mit Hilfe eines Solekühlers auf einer Temperatur von etwa 0 bis etwa 20 °C, vorzugsweise etwa 0 bis etwa 15 °C und besonders bevorzugt in einem Bereich von etwa 2 bis 10 °C gehalten.

Um ggf. weitere Verunreinigungen aus dem Produkt zu entfernen und zur Reinstmethacrylsäureestern zu gelangen, kann das Produkt noch einer adsorptiven Reinigungsstufe unterzogen werden. Es hat sich dabei beispielsweise bewährt, wenn das Reinprodukt ganz oder zumindest ein Teil des Reinproduktes mit Hilfe eines Molekularsiebes weiter gereinigt wird. Besonders saure Verunreinigungen, insbesondere im Rahmen des Herstellungsverfahrens gebildete Ameisensäure, kann so auf einfache Weise aus dem Produktstrom entfernt werden. Dabei hat es sich darüber hinaus in einigen Fällen bewährt, wenn der Produktstrom nach dem Durchlaufen der adsorptiven Reinigungsstufe noch einen oder mehrere Filter durchläuft, um ggf. im Produkt enthaltene Feststoffe zu entfernen.

Die im Rahmen der Aufarbeitung anfallenden Stoffströme umfassen überwiegend polymerisierbare Verbindungen. Um, wie bereits im Rahmen dieses Textes mehrfach beschrieben, die Ausbildung von Ruhezonen zu unterbinden, hat es sich auch im Fall des hier beschriebenen Verfahrens als vorteilhaft herausgestellt, wenn die Teile der Anlage, die mit Methacrylsäureester in Berührung kommen, ständig mit Methacrylsäureester überströmt sind. Im Rahmen einer weiteren Ausführungsform des hier vorgestellten Verfahrens wird daher ein Teilstrom an Methacrylsäureester nach dem Pufferbehälter, jedoch vor der adsorptiven Reinigungsstufe entnommen, um die Kopfbereiche derjenigen Wärmetauscher zu überspülen, welche die aus der Destillationskolonne stammenden Brüden aufnehmen.

Das im Rahmen der Reinigungsstufe gewonnene Produkt wird anschließend mit einer Temperatur in einem Bereich von etwa -5 bis etwa 20 °C, vorzugsweise etwa 0 bis etwa 15 °C und besonders bevorzugt in einem Bereich von etwa 2 bis 10 °C der Reinigungsstufe entnommen.

### Strippen der verbrauchten Säure

Im Rahmen des hier vorgestellten Verfahrens kann es beispielsweise sinnvoll sein, die bei dem Verfahren anfallende, verbrauchte Schwefelsäure einer Reinigung zu unterziehen, um sie anschließend wieder in das Verfahren zurückzuführen. Dabei kann beispielsweise ein Strom mit verbrauchter Schwefelsäure, wie er sich aus der Veresterung erhalten lässt, in einem Flotationsbehälter mit Dampf beaufschlagt werden. Dabei kann sich zumindest ein Teil der enthaltenen Feststoffe auf der Oberfläche der Flüssigkeit abscheiden, wobei diese abgeschiedenen Feststoffe ausgekreist werden können. Die Brüden werden anschließend in einem Wärmetauscher, vorzugsweise mit Wasserkühlung, kondensiert, gekühlt und in die Veresterungsreaktion zurückgeführt.

Es hat sich dabei als in einigen Fällen als vorteilhaft erwiesen, wenn zur Verminderung von Korrosion in den Wärmetauschern und zur weiteren Verbesserung der Kühlwirkung ein Gemisch aus Wasser und organischen Verbindungen, wie es im Rahmen der Veresterung bei der Reinigung des hergestellten Methacrylsäureesters durch Waschen erhalten wird, in die Wärmetauscher derart eingebracht wird, dass die Köpfe der Wärmetauscher mit diesem Gemisch besprüht werden. Neben der korrosionsvermindemden Wirkung und der Kühlung der Säure im Wärmetauscher weist diese Vorgehensweise einen weiteren Vorteil auf. Material, das aus der Veresterung stammt (ein Gemisch aus Wasser und überwiegend Methanol), wird zusammen mit der aus eben diesem Prozess stammenden Methacrylsäure und Methacrylsäureester in den Veresterungsprozess zurückgeführt. Im Stripper werden durch die oben beschriebene Flotation Gemische von Säure und Feststoffen erhalten. Diese werden nach ihrer Abtrennung einer beliebigen weiteren Verwendung oder der Entsorgung zugeführt. Möglich ist beispielsweise, das erhaltene Gemisch in einer Spaltanlage zu verbrennen und damit wieder Schwefelsäure zu erzeugen.
einen Teil der im Prozess eingesetzten Energie zurückzugewinnen.

Die beim Strippen anfallenden, nicht kondensierbaren gasförmigen Verbindungen werden einer beliebigen Weiterverwendung zugeführt oder entsorgt.

Die hier beschriebene Anlage zur Entfernung von Feststoffen aus der verbrauchten Säure sowie zur Rückführung von Material aus dem Veresterungsprozess in eben diesen Prozess kann aus Gründen der Betriebssicherheit beispielsweise auch doppelt ausgeführt sein. So können die zwei oder mehr Flotationsbehälter zeitlich versetzt eingesetzt werden. Da sich in diesen Behältern Feststoffe absetzen können, ist es vorteilhaft, diese zu entfernen, wenn der jeweilige Flotationsbehälter sich nicht im Einsatz befindet.

Weiterhin betrifft die vorliegende Erfindung die Verwendung der durch das erfindungsgemäße Verfahren erhältlichen Methacrylsäure beziehungsweise des durch das erfindungsgemäße Verfahren erhältlichen Methacrylsäureester in Fasern, Filmen, Lacken, Formmassen, Formkörpern, Papierhilfsmittel, Lederhilfsmittel, Flockungsmittel und Bohrzusätze. Zudem betrifft die vorliegende Erfindung Fasern, Filme, Lacke, Formmassen, Formkörper, Papierhilfsmittel, Lederhilfsmittel, Flockungsmittel und Bohrzusätze, die auf einer durch das erfindungsgemäße Verfahren erhältlichen Methacrylsäure beziehungsweise einem durch das erfindungsgemäße Verfahren erhältlichen Methacrylsäureester basieren.

Nachfolgend werden verschiedene Verfahrenselemente erläutert, die grundsätzlich einzeln oder als Ensemble aus zwei oder mehr der genannten Verfahrenselemente mit der vorliegenden Erfindung verbindbar sind. Gegebenenfalls kann es vorteilhaft sein, wenn die im Rahmen des vorliegenden Textes vorgestellten Verfahrenselemente derart mit der vorliegenden Erfindung kombiniert werden, dass sie insgesamt zu einem Verfahren zur Herstellung von Estern der Methacrylsäure oder einem Verfahren zur Herstellung von Methacrylsäure kombiniert werden. Es sei jedoch auch darauf hingewiesen, dass sich zumeist vorteilhafte Effekte auch dann erzielen lassen, wenn der Gegenstand der vorliegenden Erfindung als solcher in einem anderen Umfeld eingesetzt wird oder nur mit einem Teil der hier vorgestellten Verfahrenselemente kombiniert wird.

### Herstellung von Acetoncyanhydrin

In diesem Verfahrenselement wird Acetoncyanhydrin nach allgemein bekannten Verfahren (siehe beispielsweise Ullmanns Enzyklopädie der technischen Chemie, 4. Auflage, Band 7) hergestellt. Häufig werden dabei als Reaktionspartner Aceton und Blausäure eingesetzt. Bei der Umsetzung handelt es sich um eine exotherme Reaktion. Um einer Zersetzung des im Rahmen dieser Reaktion gebildeten Acetoncyanhydrin entgegenzuwirken, wird üblicherweise die Reaktionswärme durch eine geeignete Vorrichtung abgeführt. Die Umsetzung kann dabei grundsätzlich als Batchprozess oder als kontinuierliches Verfahren geführt werden, sofern eine kontinuierliche Fahrweise bevorzugt ist, wird die Umsetzung häufig in einem Schlaufenreaktor, der entsprechend eingerichtet ist, durchgeführt.

Ein Hauptmerkmal einer in hohen Ausbeuten zum gewünschten Produkt führenden Fahrweise liegt oft darin, dass bei ausreichender Reaktionszeit das Reaktionsprodukt gekühlt wird und das Reaktionsgleichgewicht in Richtung des Reaktionsproduktes verschoben wird. Darüber hinaus wird das Reaktionsprodukt zum Vorteil der Gesamtausbeute häufig mit einem entsprechenden Stabilisator versetzt, um einer Zersetzung bei der späteren Aufarbeitung in die Ausgangsstoffe vorzubeugen.

Die Vermischung der Reaktionspartner Aceton und Blausäure kann grundsätzlich auf im Wesentlichen beliebige Weise erfolgen. Die Art der Vermischung hängt insbesondere davon ab, ob eine diskrete Fahrweise, beispielsweise im Batchreaktor, oder eine kontinuierliche Fahrweise, beispielsweise im Schlaufenreaktor, gewählt wird.

Grundsätzlich kann es vorteilhaft sein, wenn das Aceton in die Reaktion über einen Vorlagebehälter eingespeist wird, der über einen Waschturm verfügt. Entlüftungsleitungen, die Aceton und Blausäure enthaltende Abluft führen, können so beispielsweise durch diesen Vorlagebehälter geführt werden. Im Waschturm, der an den Vorlagebehälter angeschlossen ist, kann die aus dem Vorlagebehälter entweichende Abluft mit Aceton gewaschen werden, wodurch Blausäure aus der Abluft entfernt und in den Prozess rückgeführt werden. Hierzu wird beispielsweise ein Teil der vom Vorlagebehälter in die Reaktion eingeführten Acetonmenge im Teilstrom über einen Kühler, vorzugsweise über einen Sole-Kühler, in den Kopf des Waschturms geführt und so das gewünschte Ergebnis erzielt.

Je nach Umfang der zu produzierenden Menge an Endprodukten kann es vorteilhaft sein, dass Aceton aus mehr als nur einem Vorlagebehälter der Reaktion zuzuführen. Dabei kann jeder der zwei oder mehr Vorlagebehälter einen entsprechenden Waschturm tragen. Es ist jedoch in vielen Fällen ausreichen, wenn nur einer der Vorlagebehälter mit einem entsprechenden Waschturm ausgerüstet ist. In diesem Fall ist es jedoch oft sinnvoll, wenn entsprechende Abluft führende Leitungen, die Aceton und Blausäure transportieren können, über diesen Behälter bzw. über diesen Waschturm geführt werden.

Die Temperatur des Acetons im Vorlagebehälter kann grundsätzlich innerhalb eines im Wesentlichen beliebigen Bereiches liegen, insofern das Aceton sich bei der entsprechenden Temperatur im flüssigen Zustand befindet. Vorteilhafterweise beträgt die Temperatur im Vorlagebehälter jedoch etwa 0 bis etwa 20° C.

Im Waschturm wird das zum Waschen eingesetzte Aceton über einem entsprechenden Kühler, beispielsweise über einen Plattenkühler mit Sole auf eine Temperatur von etwa 0 bis etwa 10° C gekühlt. Die Temperatur des Acetons beim Eintritt in den Waschturm beträgt daher vorzugsweise beispielsweise etwa 2 bis etwa 6 °C.

Die im Rahmen der Reaktion benötigte Blausäure kann entweder in flüssiger oder in gasförmiger Form in den Reaktor eingeführt werden. Es kann sich dabei beispielsweise um Rohgas aus dem BMA- oder aus dem Andrussow-Prozess handeln.

Der Cyanwasserstoff kann beispielsweise verflüssigt werden, beispielsweise durch den Einsatz einer entsprechenden Kühlsole. Anstelle von verflüssigter Blausäure kann Kokereigas eingesetzt werden. So werden beispielsweise Cyanwasserstoff enthaltende Kokereigase nach einer Wäsche mit Pottasche kontinuierlich im Gegenstrom mit Aceton, das 10 % Wasser enthält, gewaschen und die Reaktion zu Acetoncyanhydrin kann in Gegenwart eines basischen Katalysators in zwei hintereinander geschalteten Gaswaschkolonnen durchgeführt werden.

Im Rahmen einer weiteren Ausführungsform kann ein Cyanwasserstoff und inerte Gase enthaltendes Gasgemisch, insbesondere ein Rohgas aus dem BMA- oder aus dem Andrussow-Prozess mit Aceton in Gegenwart eines basischen Katalysators und Acetoncyanhydrin in einem Gas-Flüssig-Reaktor umgesetzt werden.

Im Rahmen des hier beschriebenen Verfahrens wird vorzugsweise ein BMA-Rohgas oder ein Andrussow-Rohgas eingesetzt. Dass aus den oben genannten üblichen Verfahren zur Herstellung von Cyanwasserstoff resultierende Gasgemisch kann als solches oder nach einer Säurewäsche verwendet werden. Das Rohgas aus dem BMA-Prozess, bei welchem aus Methan und Ammoniak im Wesentlichen Blausäure und Wasserstoff gebildet werden, enthält typischerweise 22,9 Vol.-% HCN, 71,8 Vol.-% H₂, 2,5 Vol.-% NH3, 1,1 Vol.-% N₂, 1,7 Vol.-% CH₄. Im bekannten Andrussow-Prozess werden aus Methan und Ammoniak und Luftsauerstoff Blausäure und Wasser gebildet. Das Rohgas des Andrussow-Prozesses enthält beim Einsatz von Sauerstoff als Sauerstoffquelle typischerweise 8 Vol.-% HCN, 22 Vol.-% H₂, 46,5 Vol.-% N₂, 15 Vol.-% H₂O, 5 Vol.-% CO, 2,5 Vol.-% NH3 und je 0,5 Vol.-% CH₄ und CO₂.

Beim Einsatz eines nicht säuregewaschenen Rohgases aus dem BMA- oder Andrussow-Prozess wirkt der im Rohgas enthaltene Ammoniak häufig als Katalysator für die Reaktion. Da der im Rohgas enthaltene Ammoniak häufig die als Katalysator erforderliche Menge übersteigt und deshalb zu hohen Verlusten an zur Stabilisierung eingesetzter Schwefelsäure führen kann, wird ein derartiges Rohgas oft einer Säurewäsche unterzogen, um Ammoniak daraus zu eliminieren. Beim Einsatz eines solchen Säure gewaschenen Rohgases muss dann allerdings ein geeigneter basischer Katalysator dem Reaktor in katalytischer Menge zugesetzt werden. Im Prinzip können dabei bekannte anorganische oder organische basische Verbindungen als Katalysator fungieren.

Cyanwasserstoff in gasförmiger oder in flüssiger Form bzw. ein Cyanwasserstoff enthaltendes Gasgemisch und Aceton werden im Rahmen der kontinuierlichen Fahrweise fortlaufend einem Schlaufenreaktor zugeführt. Der Schlaufenreaktor umfasst dabei mindestens eine Möglichkeit zum Zuführen von Aceton oder zwei oder mehr solcher Möglichkeiten, mindestens eine Möglichkeit zur Zuführung von flüssiger oder gasförmiger Blausäure, oder zwei oder mehr solcher Möglichkeiten sowie mindestens eine Möglichkeit zum Zuführen eines Katalysators.

Als Katalysator eignen sich grundsätzlich beliebige alkalische Verbindungen wie Ammoniak, Natronlauge oder Kalilauge, die die Umsetzung von Aceton und Blausäure zu Acetoncyanhydrin katalysieren können. Es hat sich jedoch als vorteilhaft herausgestellt, wenn als Katalysator ein organischer Katalysator, insbesondere ein Amin eingesetzt wird. Geeignet sind beispielsweise sekundäre oder tertiäre Amine, wie Dimethylamin, Dipropylamin, Triethylamin, Tri-n-propylamin und dergleichen.

Ein im Rahmen des beschriebenen Verfahrenselementes einsetzbarer Schlaufenreaktor weist darüber hinaus noch mindestens eine Pumpe, oder zwei oder mehr Pumpen, und mindestens eine Mischvorrichtung, oder zwei oder mehr solcher Mischvorrichtungen auf.

Als Pumpe eignen sich grundsätzlich alle Pumpen, die geeignet sind, den Umlauf des Reaktionsgemisches im Schlaufenreaktor zu gewährleisten.

Als Mischvorrichtungen sind sowohl Mischvorrichtungen mit beweglichen Elementen als auch so genannte Statikmischer geeignet, bei denen unbewegliche Strömungswiderstände vorgesehen sind. Im Falle des Einsatzes von Statikmischern sind beispielsweise solche geeignet, die einen Betriebsübertrug von mindestens etwa 10, beispielsweise mindestens etwa 15 oder mindestens etwa 20 bar unter Betriebsbedingungen ohne wesentliche Einschränkungen der Funktionsfähigkeit erlauben. Entsprechende Mischer können aus Kunststoff oder Metall bestehen. Als Kunststoff eignen sich beispielsweise PVC, PP; HDPE, PVDF, PFA oder PTFE. Metallmischer können beispielsweise aus Nickellegierungen, Zirkonium, Titan und dergleichen bestehen. Ebenfalls geeignet sind beispielsweise Rechteckmischer.

Die Zugabe des Katalysators erfolgt vorzugsweise im Schlaufenreaktor nach der Pumpe und vor einem im Schlaufenreaktor vorhandenen Mischelement. Katalysatoren werden im Rahmen der beschriebenen Reaktion beispielsweise in einer solchen Menge eingesetzt, dass die Gesamtreaktion bei einem pH-Wert von maximal 8, insbesondere maximal etwa 7,5 oder etwa 7 gefahren wird. Es kann bevorzugt sein, wenn der pH-Wert bei der Reaktion sich innerhalb eines Rahmens von etwa 6, 5 bis etwa 7, 5, beispielsweise etwa 6,8 bis etwa 7,2 bewegt.

Es ist im Rahmen des beschriebenen Verfahrens alternativ zur Zugabe des Katalysators in den Schlaufenreaktor nach der Pumpe und vor einer Mischvorrichtung auch möglich, den Katalysator zusammen mit dem Aceton in den Schlaufenreaktor einzuspeisen. In einem solchen Fall kann es vorteilhaft sein, wenn für eine entsprechende Vermischung von Aceton und Katalysator vor Einspeisung in den Schlaufenreaktor gesorgt wird. Eine entsprechende Vermischung kann beispielsweise durch den Einsatz eines Mischers mit beweglichen Teilen oder durch Einsatz eines Statikmischers erfolgen.

Wenn im Rahmen des beschriebenen Verfahrens eine kontinuierliche Fahrweise in einem Schlaufenreaktor als Betriebsart gewählt wird, so kann es zweckmäßig sein, den Zustand des Reaktionsgemisches durch punktuelle oder fortlaufende Analysen zu untersuchen. Dies bietet den Vorteil, dass gegebenenfalls auch auf Zustandsänderungen im Reaktionsgemisch schnell reagiert werden kann. Darüber hinaus lassen sich so beispielsweise die Reaktionspartner möglichst genau dosieren, um Ausbeuteverluste zu minimieren.

Eine entsprechende Analytik kann beispielsweise durch Probennahme in der Reaktorschlaufe erfolgen. Geeignete Analyseverfahren sind beispielsweise pH-Messung, Messung der Wärmetönung oder Messung der Zusammensetzung des Reaktionsgemisches durch geeignete spektroskopische Verfahren.

Insbesondere im Rahmen der Umsatzkontrolle, Qualitätsaspekten und Sicherheit hat es sich häufig bewährt, den Umsatz im Reaktionsgemisch über die aus dem Reaktionsgemisch abgeführte Wärme zu bestimmen und mit der theoretisch frei werdenden Wärme zu vergleichen.

Die eigentliche Reaktion kann bei geeigneter Wahl des Schlaufenreaktors grundsätzlich in den innerhalb des Schlaufenreaktors angeordneten Rohrsystemen erfolgen. Da die Reaktion jedoch exotherm ist, sollte, um Ausbeuteverlust zu vermeiden, auf eine ausreichende Kühlung bzw. auf eine ausreichende Abfuhr der Reaktionswärme geachtet werden. Es hat sich häufig als vorteilhaft erwiesen, wenn die Reaktion innerhalb eines Wärmetauschers, vorzugsweise innerhalb eines Rohrbündelwärmetauschers abläuft. Je nach zu produzierender Produktmenge kann die Kapazität eines entsprechenden Wärmetauschers unterschiedlich gewählt werden. Für großtechnische Verfahren haben sich insbesondere Wärmetauscher mit einem Volumen von etwa 10 bis etwa 40 m³ als besonders geeignet erwiesen. Bei den bevorzugt eingesetzten Rohrbündelwärmetauschern handelt es sich um Wärmetauscher die in einen flüssigkeitsdurchströmten Mantel ein flüssigkeitsdurchströmtes Rohrbündel aufweisen. Je nach Rohrdurchmesser, Packungsdichte, etc. kann der Wärmeübergang zwischen den beiden Flüssigkeiten entsprechend eingestellt werden. Es ist im Rahmend es beschriebenen Verfahrens grundsätzlich möglich, die Reaktion dahingehend zu führen, dass das Reaktionsgemisch durch den Wärmetauscher im Rohrbündel selbst gefahren wird und die Reaktion innerhalb des Rohrbündels stattfindet, wobei die Wärme aus dem Rohrbündel in die Mantelflüssigkeit abgeführt wird.

Es hat sich jedoch ebenfalls als praktikabel und in vielen Fällen als sinnvoll erwiesen, das Reaktionsgemisch durch den Mantel des Wärmetauschers zu führen, während die zur Kühlung eingesetzte Flüssigkeit innerhalb des Rohrbündels zirkuliert. Dabei hat es sich in vielen Fällen als vorteilhaft erwiesen, wenn die Reaktionsmischung im Mantel zur Erzielung einer höheren Verweildauer und einer besseren Durchmischung über Strömungswiderstände, vorzugsweise Umlenkbleche, verteilt wird.

Das Verhältnis von Mantelvolumen zum Volumen des Rohrbündels kann dabei, je nach Auslegung des Reaktors, etwa 10 zu 1 bis etwa 1 zu 10 betragen, vorzugsweise ist das Volumen des Mantels größer als das Volumen des Rohrbündels (bezogen auf den Inhalt der Rohre).

Die Wärmeabfuhr aus dem Reaktor wird mit einem entsprechenden Kühlmittel, beispielsweise mit Wasser, so eingestellt, dass die Reaktionstemperatur innerhalb eines Korridors etwa 25 bis etwa 45 °C, insbesondere bei etwa 30 bis etwa 38, insbesondere bei etwa 33 bis etwa 35 °C liegt.

Aus dem Schlaufenreaktor wird kontinuierlich ein Produkt abgeführt. Das Produkt weist eine Temperatur im Rahmen der oben genannten Reaktionstemperaturen, beispielsweise eine Temperatur von etwa 35 °C auf. Das Produkt wird über einen oder mehrere Wärmetauscher, insbesondere über einen oder mehrere Plattenwärmetauscher gekühlt. Dabei kommt beispielsweise eine Solekühlung zum Einsatz. Die Temperatur des Produktes nach Kühlung soll etwa 0 bis 10, insbesondere 1 bis etwa 5 °C betragen. Das Produkt wird vorzugsweise in einen Lagerbehälter überführt, der eine Pufferfunktion aufweist. Zusätzlich kann das Produkt im Lagerbehälter beispielsweise durch ständiges Abführen eines Teilstroms aus dem Lagerbehälter zu einem geeigneten Wärmetauscher, beispielsweise zu einem Plattenwärmetauscher, weiter gekühlt werden, bzw. auf einer geeigneten Lagertemperatur gehalten werden. Es ist durchaus möglich, dass in dem Lagerbehälter eine Nachreaktion stattfinden kann.

Die Rückführung des Produktes in den Lagerbehälter, kann grundsätzlich auf beliebige Weise erfolgen. Es hat sich jedoch in einigen Fällen als vorteilhaft herausgestellt, dass das Produkt derart über ein System aus einer oder mehreren Düsen in den Lagerbehälter rückgeführt wird, dass innerhalb des Lagerbehälters eine entsprechende Durchmischung des gelagerten Produktes stattfindet.

Aus dem Lagerbehälter wird weiterhin kontinuierlich Produkt in einen Stabilisierungsbehälter abgeführt. Dort wird das Produkt mit einer geeigneten Säure, beispielsweise mit H₂SO₄, versetzt. Dabei wird der Katalysator deaktiviert und das Reaktionsgemisch auf einen pH-Wert von etwa 1 bis etwa 3, insbesondere etwa 2 eingestellt. Als Säure eignet sich insbesondere Schwefelsäure, beispielsweise Schwefelsäure mit einem Gehalt von etwa 90 bis etwa 105 %, insbesondere von etwa 93 bis etwa 98 % H₂SO₄.

Das stabilisierte Produkt wird dem Stabilisierungsbehälter entnommen und in die Aufreinigungsstufe überführt. Dabei kann ein Teil des entnommenen, stabilisierten Produktes beispielsweise derart in den Stabilisierungsbehälter zurückgeführt werden, dass eine ausreichende Durchmischung des Behälters über ein System aus einer oder mehreren Düsen gewährleistet ist.

### ACH-Aufarbeitung

Im Rahmen eines weiteren Verfahrenselementes, das im Zusammenhang mit der vorliegenden Erfindung eingesetzt werden kann, wird Acetoncyanhydrin, das in einer vorgelagerten Stufe beispielsweise aus der Umsetzung von Aceton mit Blausäure erhalten wurden, einer destillativen Aufarbeitung unterzogen. Dabei wird das stabilisierte rohe Acetoncyanhydrin über eine entsprechende Kolonne von niedrigsiedenden Bestandteilen befreit. Ein geeignetes Destillationsverfahren kann beispielsweise über nur eine Kolonne geführt werden. Es ist jedoch ebenfalls möglich, im Rahmen einer entsprechenden Aufreinigung von rohem Acetoncyanhydrin eine Kombination von zwei oder mehr Destillationskolonnen auch kombiniert mit einem Fallfilmverdampfer einzusetzen. Weiterhin könne zwei oder mehrere Fallfilmverdampfer oder auch zwei oder mehrere Destillationskolonnen miteinander kombiniert werden.

Das rohe Acetoncyanhydrin kommt in der Regel mit einer Temperatur von etwa 0 bis etwa 15 °C, beispielsweise einer Temperatur von etwa 5 bis etwa 10 °C aus der Lagerung zur Destillation. Grundsätzlich kann das rohe Acetoncyanhydrin direkt in die Kolonne eingeführt werden. Es hat sich jedoch in einigen Fällen bewährt, wenn zunächst das rohe, kühle Acetoncyanhydrin über einen Wärmetauscher einen Teil der Wärme des bereits destillativ gereinigten Produktes übernimmt. Daher wird im Rahmen einer weiteren Ausführungsform des hier beschriebenen Verfahrens das rohe Acetoncyanhydrin über einen Wärmetauscher auf eine Temperatur von etwa 60 bis 80 °C erhitzt.

Die destillative Reinigung des Acetoncyanhydrin erfolgt über eine Destillationskolonne mit mehr als 10 Böden oder über eine Kaskade von zwei oder mehr entsprechend geeigneter Destillationskolonnen. Die Beheizung des Kolonnensumpfes erfolgt vorzugsweise mit Dampf. Es hat sich als vorteilhaft herausgestellt, wenn die Sumpftemperatur eine Temperatur von 140 °C nicht übersteigt, gute Ausbeuten und eine gute Reinigung haben sich erzielen lassen, wenn die Sumpftemperatur nicht größer als etwa 130 °C oder nicht höher als etwa 110 °C ist. Die Temperaturangaben beziehen sich dabei auf die Wandtemperatur des Kolonnensumpfes.

Das rohe Acetoncyanhydrin wird im oberen Drittel der Kolonne dem Kolonnenkörper zugeführt. Die Destillation wird vorzugsweise bei verringertem Druck, beispielsweise bei einem Druck von etwa 50 bis etwa 900 mbar, insbesondere etwa 50 bis etwa 250 mbar und mit guten Ergebnissen zwischen 50 bis etwa 150 mbar durchgeführt.

Am Kopf der Kolonne werden gasförmige Verunreinigungen, insbesondere Aceton und Blausäure, entnommen, die abgetrennten gasförmigen Stoffe werden über einen Wärmetauscher oder eine Kaskade von zwei oder mehr Wärmetauschern gekühlt. Hierbei wird vorzugsweise eine Solekühlung mit einer Temperatur von etwa 0 bis etwa 10 °C eingesetzt. Dabei wird den gasförmigen Inhaltsstoffen der Brüden die Gelegenheit gegeben, zu kondensieren. Die erste Kondensationsstufe kann beispielsweise bei Normaldruck stattfinden. Es ist jedoch ebenso möglich und hat sich in einigen Fällen als vorteilhaft erwiesen, wenn diese erste Kondensationsstufe unter verringertem Druck, vorzugsweise bei dem Druck, der im Rahmen der Destillation vorherrscht, erfolgt. Das Kondensat wird in einen gekühlten Auffangbehälter weitergeleitet und dort bei einer Temperatur von etwa 0 bis etwa 15 °C, insbesondere bei etwa 5 bis etwa 10 °C gesammelt.

Die im Rahmen des ersten Kondensationsschrittes nicht kondensierenden gasförmigen Verbindungen werden über eine Vakuumpumpe aus dem Unterdruckraum entfernt. Hierbei ist grundsätzlich eine beliebige Vakuumpumpe einsetzbar. Es hat sich jedoch in vielen Fällen als vorteilhaft erwiesen, wenn eine Vakuumpumpe eingesetzt wird, die aufgrund ihrer Bauweise nicht zum Eintrag flüssiger Verunreinigungen in den Gasstrom führt. Vorzugsweise werden hier daher beispielsweise trocken laufende Vakuumpumpen eingesetzt.

Der auf der Druckseite der Pumpe entweichende Gasstrom wird über einen weiteren Wärmetauscher geführt, der vorzugsweise mit Sole bei einer Temperatur von etwa 0 bis etwa 15 °C gekühlt wird. Hierbei kondensierende Inhaltsstoffe werden ebenfalls in dem Sammelbehälter gesammelt, der bereits die unter Vakuumbedingungen gewonnenen Kondensate auffängt. Die auf der Druckseite der Vakuumpumpe durchgeführte Kondensation kann beispielsweise durch einen Wärmetauscher, jedoch auch mit einer Kaskade von zwei oder mehr seriell parallel angeordneten Wärmetauschern erfolgen. Nach diesem Kondensationsschritt verbleibende gasförmigen Stoffen werden abgeführt und einer beliebigen weiteren Verwertung, beispielsweise einer thermischen Verwertung, zugeführt.

Die gesammelten Kondensate können ebenfalls beliebig weiterverwertet werden. Es hat sich jedoch als unter ökonomischen Gesichtspunkten äußerst Vorteilhaft erwiesen, die Kondensate in die Reaktion zur Herstellung von Acetoncyanhydrin zurückzuführen. Dies erfolgt vorzugsweise an einer oder mehreren Stellen, die Zugang zum Schlaufenreaktor ermöglichen. Die Kondensate können grundsätzlich eine beliebige Zusammensetzung aufweisen, sofern sie die Herstellung des Acetoncyanhydrins nicht stören. In vielen Fällen wird die überwiegende Menge des Kondensates jedoch aus Aceton und Blausäure bestehen, beispielsweise in einem molaren Verhältnis von etwa 2:1 bis etwa 1:2, häufig in einem Verhältnis von etwa 1:1.

Das aus dem Sumpf der Destillationskolonne gewonnene Acetoncyanhydrin wird zunächst über einen ersten Wärmetauscher durch das zugeführte, kalte rohe Acetoncyanhydrin auf eine Temperatur von etwa 40 bis etwa 80 °C abgekühlt. Anschließend wird das Acetoncyanhydrin über einen mindestens einen weiteren Wärmetauscher auf eine Temperatur von etwa 30 bis etwa 35 °C gekühlt und ggf. zwischengelagert.

### Amidierung

Im Rahmen eines weiteren Verfahrenselements, wie er häufig bei der Herstellung von Methacrylsäure oder von Estern der Methacrylsäure vorgesehen ist, wird Acetoncyanhydrin einer Hydrolyse unterzogen. Dabei bildet sich bei verschiedenen Temperaturstufen nach einer Reihe von Reaktionen als Produkt Methacrylamid.

Die Umsetzung wird in einer dem Fachmann bekannten Weise durch eine Reaktion zwischen konzentrierter Schwefelsäure und Acetoncyanhydrin bewirkt. Die Umsetzung ist exotherm, so dass in vorteilhafter Weise Reaktionswärme aus dem System abgeführt wird.

Die Umsetzung kann auch hier wieder im Batchverfahren oder in kontinuierlichen Verfahren durchgeführt werden. Letzteres hat sich in vielen Fällen als vorteilhaft erwiesen. Sofern die Umsetzung im Rahmen eines kontinuierlichen Verfahrens durchgeführt wird, hat sich der Einsatz von Schlaufenreaktoren bewährt. Die Umsetzung kann beispielsweise in nur einem Schlaufenreaktor erfolgen. Es kann jedoch vorteilhaft sein, wenn die Umsetzung in einer Kaskade von zwei oder mehreren Schlaufenreaktoren durchgeführt wird.

Ein geeigneter Schlaufenreaktor weist im Rahmen des beschriebenen Verfahrens eine oder mehrere Zufuhrstellen für Acetoncyanhydrin, eine oder mehrere Zufuhrstellen für konzentrierte Schwefelsäure, einen oder mehrere Gasabscheider, einen oder mehrere Wärmetauscher und eine oder mehrere Mischer auf.

Die Hydrolyse von Acetoncyanhydrin mit Schwefelsäure zu Methacrylamid ist, wie bereits beschrieben, exotherm. Die im Rahmen der Reaktion anfallende Reaktionswärme muss dem System jedoch zumindest weitgehend entzogen werden, da mit zunehmender Temperatur bei der Umsetzung die Ausbeute sinkt. Zwar ist es grundsätzlich möglich mit entsprechenden Wärmetauschern eine schnelle und umfassende Abfuhr der Reaktionswärme zu erzielen. Es kann jedoch auch nachteilig sein, das Gemisch zu sehr zu kühlen, da für einen entsprechenden Austausch an den Wärmetauschern ein ausreichender Wärmeübergang erforderlich ist. Da mit sinkender Temperatur die Viskosität des Gemischs stark ansteigt, wird so einerseits die Zirkulation im Schlaufenreaktor erschwert, andererseits lässt sich eine ausreichende Abfuhr der Reaktionsenergie aus dem System nicht mehr gewährleisten.

Darüber hinaus können zu geringe Temperaturen im Reaktionsgemisch zu einer Kristallisation von Inhaltsstoffen des Reaktionsgemischs an den Wärmetauschern führen. Dadurch wird der Wärmeübergang weiter verschlechtert, wodurch sich eindeutiger Ausbeuterückgang verzeichnen lässt. Darüber hinaus kann der Schlaufenreaktor nicht mit den optimalen Mengen an Reaktanden beschickt werden, so dass insgesamt die Effizienz des Verfahrens leidet.

Im Rahmen einer Ausgestaltung des Verfahrens wird aus einem Strom von Acetoncyanhydrin ein Teil, vorzugsweise etwa zwei Drittel bis etwa drei Viertel, des Volumenstroms in einen ersten Schlaufenreaktor eingeführt. Vorzugsweise weist ein erster Schlaufenreaktor einen oder mehrere Wärmetauscher, eine oder mehrere Pumpen, einen oder mehrere Mischelemente und einen oder mehrere Gasabscheider auf. Die den ersten Schlaufenreaktor durchlaufenden Umwälzströme liegen vorzugsweise im Bereich von etwa 100 bis 450 m³/h, bevorzugt in einem Bereich von 200 bis 400 m³/h und darüber hinaus bevorzugt in einem Bereich von etwa 250 bis 350 m³/h. In einen auf den ersten Schlaufenreaktor folgenden mindestens einen weiteren Schlaufenreaktor liegen die Umwälzströme vorzugsweise in einem Bereich von etwa 40 bis 450 m³/h, bevorzugt in einem Bereich von 50 bis 400 m³/h und darüber hinaus bevorzugt in einem Bereich von etwa 60 bis 350 m³/h. Weiterhin sind als Temperaturdifferenz über den Wärmetauscher etwa 1 bis 10 °C bevorzugt, wobei etwa 2 bis 7 °C besonders bevorzugt sind.

Die Zufuhr des Acetoncyanhydrin kann grundsätzlich an beliebiger Stelle in den Schlaufenreaktor erfolgen. Es hat sich jedoch als vorteilhaft erwiesen, wenn die Zufuhr in ein Mischelement, beispielsweise in einen Mischer mit beweglichen Teilen oder einen Statikmischer erfolgt. Die Zufuhr der Schwefelsäure erfolgt vorteilhafter Weise vor der Acetoncyanhydrin-Zugabe. Ansonsten ist es jedoch ebenfalls möglich, die Schwefelsäure an beliebiger Stelle in den Schlaufenreaktor einzuführen.

Das Verhältnis der Reaktanden im Schlaufenreaktor wird so gesteuert, dass ein Überschuss Schwefelsäure vorliegt. Der Überschuss an Schwefelsäure beträgt, bezüglich des molaren Verhältnisses der Inhaltsstoffe, im ersten Schlaufenreaktor etwa 1,8:1 bis etwa 3:1 und im letzten Schlaufenreaktor etwa 1,3:1 bis etwa 2:1.

In einigen Fällen hat es sich als vorteilhaft erwiesen, mit einem derartigen Überschuss an Schwefelsäure die Reaktion im Schlaufenreaktor zu betreiben. Die Schwefelsäure kann hier beispielsweise als Lösemittel dienen und die Viskosität des Reaktionsgemischs niedrig halten, wodurch eine höhere Abfuhr von Reaktionswärme und eine niedrigere Temperatur des Reaktionsgemischs gewährleistet werden kann. Dies kann deutliche Ausbeutevorteile mit sich bringen. Die Temperatur im Reaktionsgemisch beträgt etwa 90 bis etwa 120 °C.

Die Wärmeabfuhr wird durch einen oder mehrere Wärmetauscher im Schlaufenreaktor gewährleistet. Es hat sich dabei als vorteilhaft erwiesen, wenn die Wärmetauscher über eine geeignete Sensorik zum Einstellen der Kühlleistung verfügen, um eine zu starke Kühlung des Reaktionsgemischs aus den oben genannten Gründen zu verhindern. So kann es beispielsweise vorteilhaft sein, den Wärmeübergang im Wärmetauscher oder in den Wärmetauschern punktförmig oder kontinuierlich zu messen und daran die Kühlleistung der Wärmetauscher anzupassen. Dies kann beispielsweise über das Kühlmittel selbst geschehen. Es ist auch ebenso möglich durch entsprechende Variation der Zugabe der Reaktionspartner und durch die Erzeugung von mehr Reaktionswärme eine entsprechenden Erhitzung des Reaktionsgemischs zu erreichen. Auch eine Kombination von beiden Möglichkeiten ist denkbar. Der Schlaufenreaktor sollte darüber hinaus über mindestens einen Gasabscheider verfügen. Über den Gasabscheider wird einerseits dem Schlaufenreaktor kontinuierlich gebildetes Produkt entnommen. Andererseits lassen sich im Rahmen der Reaktion gebildete Gase so dem Reaktionsraum entziehen. Als Gas bildet sich hauptsächlich Kohlenmonoxid. Das aus dem Schlaufenreaktor entnommene Produkt wird vorzugsweise in einen zweiten Schlaufenreaktor überführt. In diesen zweiten Schlaufenreaktor wird das Schwefelsäure und Metharcylsäureamid beinhaltende Reaktionsgemisch wie es durch die Reaktion im ersten Schlaufenreaktor erhalten wurde, mit dem verbleibenden Teilstrom an Acetoncyanhydrin umgesetzt. Hierbei reagiert der Überschuss an Schwefelsäure aus dem ersten Schlaufenreaktor, oder zumindest ein Teil der überschüssigen Schwefelsäure, mit dem Acetoncyanhydrin unter weiterer Bildung von Methacrylsäureamid. Die Durchführung der Reaktion in zwei oder mehr Schlaufenreaktoren weist den Vorteil auf, dass aufgrund des Schwefelsäureüberschusses im ersten Schlaufenreaktor die Pumpbarkeit des Reaktionsgemischs und damit der Wärmeübergang und letztendlich die Ausbeute verbessert werden. Im zweiten Schlaufenreaktor ist wiederum mindestens ein Mischelement, mindestens ein Wärmetauscher und mindestens ein Gasabscheider angeordnet. Die Reaktionstemperatur im zweiten Schlaufenreaktor beträgt ebenfalls etwa 90 bis etwa 120 °C.

Das Problem der Pumpbarkeit des Reaktionsgemischs, des Wärmeübergangs und einer möglichst geringen Reaktionstemperatur stellt sich im jedem weiteren Schlaufenreaktor genauso wie im ersten. Deshalb verfügt vorteilhafter Weise auch der zweite Schlaufenreaktor über einen Wärmetauscher, dessen Kühlleistung durch entsprechende Sensorik geregelt werden kann.

Die Zufuhr des Acetoncyanhydrins erfolgt wiederum in einem geeigneten Mischelement, vorzugsweise in einen Statikmischer.

Aus dem Gasabscheider des zweiten Schlaufenreaktors wird das Produkt entnommen und zur Vervollständigung der Umsetzung und zur Bildung des Methacrylsäureamids auf eine Temperatur von etwa 140 bis etwa 180 °C erhitzt.

Die Erhitzung wird vorzugsweise derart durchgeführt, dass die Maximaltemperatur nur für einen möglichst kurzen Zeitraum, beispielsweise für eine Zeit von etwa einer Minute bis etwa 30 Minuten, insbesondere für eine Zeit von etwa zwei bis etwa acht oder etwa drei bis etwa fünf Minuten erreicht wird. Dies kann grundsätzlich in beliebigen Apparaturen zur Erzielung einer derartigen Temperatur für einen derart kurzen Zeitraum erfolgen. Beispielsweise kann die Energiezufuhr auf konventionellem Wege durch elektrische Energie oder durch Dampf erfolgen. Es ist jedoch ebenso möglich, die Energie durch elektromagnetische Strahlung, beispielsweise durch Mikrowellen zuzuführen.

Es hat sich in verschiedenen Fällen als vorteilhaft erwiesen, wenn der Erhitzungsschritt in einem Wärmetauscher mit zwei oder mehrstufiger Anordnung von Rohrwendeln, die vorzugsweise in einer mindestens doppelten, gegenläufigen Anordnung vorliegen können, erfolgt. Dabei wird das Reaktionsgemisch schnell auf eine Temperatur von etwa 140 bis 180 °C erhitzt.

Der Wärmetauscher kann beispielsweise mit einem oder mehreren Gasabscheidem kombiniert werden. So ist es beispielsweise möglich, das Reaktionsgemisch nach Verlassen der ersten Rohrwendel im Wärmetauscher durch einen Gasabscheider zu führen. Dabei können beispielsweise während der Reaktion entstehende, gasförmige Komponenten aus dem Reaktionsgemisch abgetrennt werden. Es ist ebenso möglich, das Reaktionsgemisch nach Verlassen der zweiten Wendel mit einem Gasabscheider zu behandeln. Es kann sich darüber hinaus als vorteilhaft erweisen, an beiden Stellen, sowohl nach Verlassen der ersten als auch nach Verlassen der zweiten Rohrwendel das Reaktionsgemisch mit einem Gasabscheider zu behandeln.

Die so erhältliche Amidlösung weist in der Regel eine Temperatur von mehr als 100 °C, üblicherweise eine Temperatur von etwa 140 bis 180 °C auf.

Die im Rahmen der Amidierung anfallenden gasförmigen Verbindungen können grundsätzlich in beliebiger Weise entsorgt oder einer Weiterverarbeitung zugeführt werden. Es kann in einigen Fällen jedoch vorteilhaft sein, wenn die entsprechenden Gase in einer Transportleitung dergestalt zusammengeführt werden, dass sie entweder kontinuierlich oder bei Bedarf ggf. mit Druck, beispielsweise mit Dampfdruck, beaufschlagt und so weitertransportiert werden können.

Das Vorstehende wird nunmehr anhand von nicht limitierenden Zeichnungen beispielhaft erläutert. Es zeigen:
Fig. 1: einen Anlagenverbund zur Herstellung und Verarbeitung von mit Methacrylsäure bzw. Methylmethacrylat,
Fig. 2: schematisch eine Anlage zur Herstellung von Acetoncyanhydrin,
Fig. 3: schematisch eine Aufarbeitungsanlage von Acetoncyanhydrin,
Fig. 4: schematisch Amidierungsanlage,
Fig. 5: schematisch eine Veresterungsanlage,
Fig. 6: schematisch eine Anlage zur Vorreinigung des Esters,
Fig. 7: die Feinreinigungsanlage des Esters.

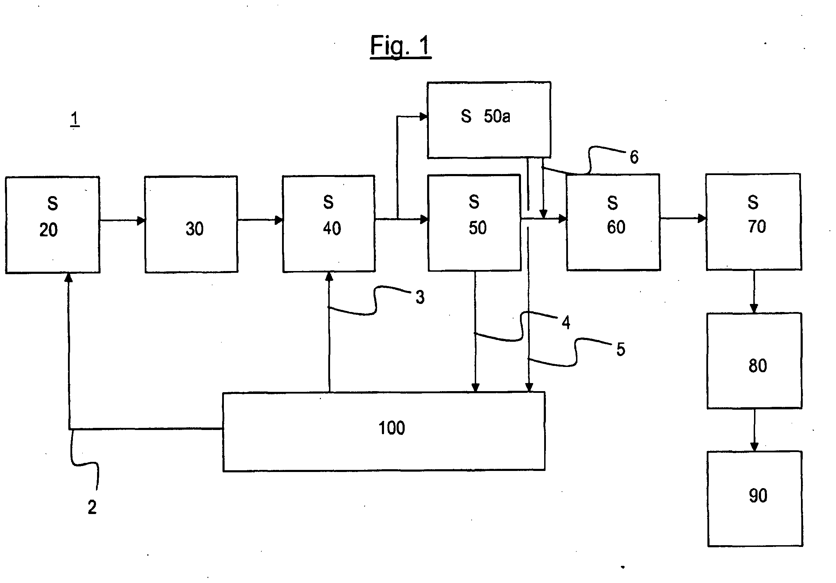

In Fig. 1 sind die bevorzugten Elemente einen Anlagenverbund 1 zur Herstellung von Methacrylsäure bzw. Methacrylsäureestern und deren Weiterverarbeitungsprodukten gezeigt. Der Anlagenverbund 1 weist verschiedene untereinander meist fluidleitenden verbundene Anlagen als Elemente dieses Verbunds auf. Zu diesem Anlagenverbund gehört die Acetoncyanhydrinherstellung 20, gefolgt von der Acetoncyanhydrinaufarbeitung 30, gefolgt von einer Amidierung 40, gefolgt von einer Veresterung/Hydrolyse 50/50a), gefolgt von einer Aufarbeitung für Ester oder Methacrylsäure 60 wiederum gefolgt von einer Feinreinigung 70, nach der der Ester, meist Methylmethacrylat, oder Methacrylsäure vorliegt. Der so gewonnene Reinester/Reinsäure kann einer Weiterverarbeitungsanlage 80 zugeführt werden. Als Weiterverarbeitungsanlagen 80 kommen vor allem Polymerisationsvorrichtungen und Reaktoren für weitere organische Reaktionen in Betracht. In den Polymerisationsreaktoren können Polymethacrylate hergestellt werden und in den Reaktoren für organische Reaktionen können die hier gewonnenen reinen Monomere zu weiteren organischen Verbindungen umgesetzt werden. Auf die Weiterverarbeitungsanlage oder die Weiterverarbeitungsanlagen 80 folgt eine Konfektionierung 90. Sofern es sich bei den Weiterverarbeitungsprodukten um Polymere aus der Methacrylsäure oder Methacrylsäureester, insbesondere Methylmethacrylat, handelt werden diese zu Fasern, Formmassen, insbesondere Granulat, Folien, Platten, Automobilteile und anderen Formkörpem durch geeignete Geräte wie Extruder, Blasextruder, Spritzgussgeräte, Spinndüsen und dergleichen, weiterverarbeitet. Weiterhin beinhaltet der Anlagenverbund 1 in vielen Fällen eine Schwefelsäureanlage 100. Hierbei kommen grundsätzlich alle dem Fachmann hierfür geeignet erscheinenden Schwefelsäureanlagen in Betracht. Beispielsweise sei auf die in Kapitel 4, Seite 89 ff. in "Integrated Pollution Prevention and Control - Draft Reference Document on Best Avalible Techniques for the Manufacture of Large Volume Inorganic Chemicals - Amonia Acids and Fertelizers", erhältlich über die Europäische Komission, in diesem Zusammenhang verwiesen. Die Schwefelsäureanlage 100 ist mit einer Reihe anderer Anlagen verbunden. So wird die Acetoncyanhydrinherstellung 20 über eine Schwefelsäureleitung 2 mit konzentrierter Schwefelsäure versorgt. Außerdem besteht eine weitere Schwefelsäureleitung 3 zwischen der Schwefelsäureanlage 100 und der Amidierung 40. Die auch als "Spent Acid" bezeichnete verdünnte Schwefelsäure aus der Veresterung 50 (Hydrolyse 50a) wird zu der Schwefelsäureanlage 100 durch die Leitungen für verbrauchte Schwefelsäure 4 bzw. 5 überführt. In der Schwefelsäureanlage 100 kann die verdünnte Schwefelsäure aufgearbeitet werden. Die Aufarbeitung der verdünnten Schwefelsäure kann beispielsweise wie in WO 02/23088 A1 oder WO 02/23089 A1 beschrieben erfolgen. Im Allgemeinen sind die Anlagen aus dem Fachmann geläufigen und für die jeweiligen Beanspruchungen geeignet erscheinenden Materialien ausgeführt. Meist handelt es sich hierbei um Edelstahl, der insbesondere eine besondere Säurefestigkeit besitzen muss. Die Bereiche der Anlagen, die mit Schwefelsäure und insbesondere mit konzentrierter Schwefelsäure betrieben werden, sind darüber hinaus mit keramischen Materialien oder Kunststoffen ausgekleidet und geschützt. Zudem kann die in der Methacrylsäureanlage 50a erhaltene Methacrylsäure über eine Methacrylsäureleitung 6 der Vorreinigung 60 zugeführt werden. Weiterhin hat es sich bewährt, dass in der Acetoncyanhydrinherstellung 20, der Amidierung 40, der Veresterung 50, der Hydrolyse 50a, der Vorreinigung 60 und auch der Endreinigung 70 einen mit "S" gekennzeichneten Stabilisators zuzusetzen.

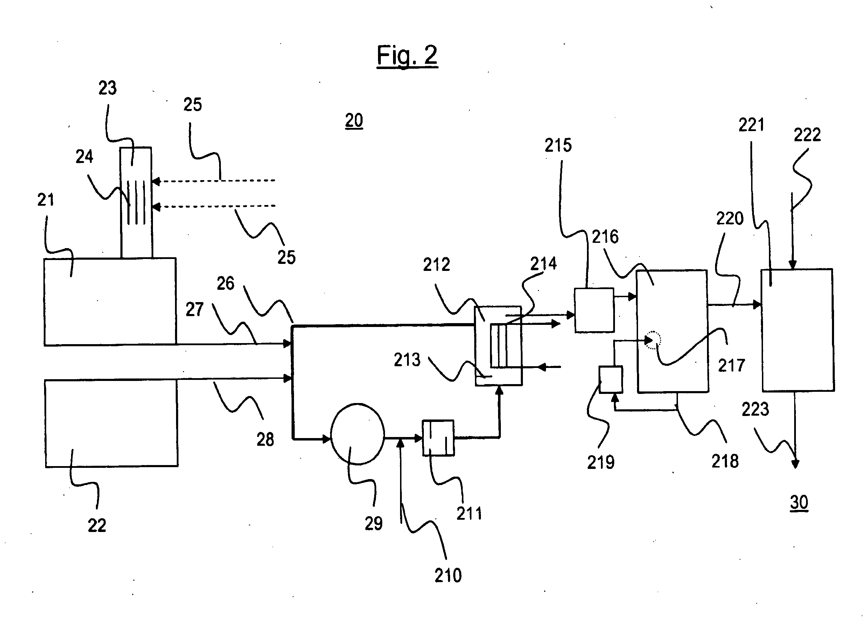

In der in Figur 2 dargestellten Acetoncyanhydrinherstellung 20 wird das Aceton in einem Acetonbehälter 21 und die Blausäure in einem Blausäurebehälter 22 bereitgestellt. Der Acetonbehälter 21 weist einen Waschturm 23 auf, der in seinem oberen Bereich einen oder mehrere Kühlelemente 24 aufweist. In den Waschturm 23 mündet eine Reihe von Abgasleitungen 25, die aus verschiedenen Anlagen des Anlagenverbunds 1 stammen. In einen Schlaufenreaktor 26 wird das Aceton über die Acetonzuleitung 27 und die Blausäure über die Blausäurezuleitung 28 eingespeist stromab der Blausäurezuleitung 28 befindet sich eine Pumpe 29, wiederum gefolgt von einer Katalysatoreinspeisung 210, auf die ein statischer Mischer 211 folgt. Hiernach schließt sich ein Wärmetauscher 212 an, der eine Reihe von Strömungswiderständen 213 und mindestens eine Kühlleitung 214 aufweist. In dem Schlaufenreaktor 26 wird das aus Aceton, Blausäure und Katalysator bestehende Reaktionsgemisch zu einem erheblichen Teil in einem Kreislauf gefahren, der durch fette Linien gekennzeichnet ist. Aus dem Wärmetauscher 212 wird die Reaktionsmischung über die Strömungswiderstände entlang der Kühlleitungen 214 geführt und ein Teil des Kreislaufstromes in einen weiteren Wärmetauscher 215 geleitet, an den sich ein Sammelbehälter 216 anschließt, in dem sich eine Düse 217 als Teil eines Kühlkreislaufs 218 mit einem Wärmetauscher 219 befindet, wodurch das Reaktionsprodukt zum einen in Bewegung und zum anderen kühl gehalten wird. Über eine sich an den Sammelbehälter 216 anschließende Ableitung 220 ist ein Stabilisierbehälter 221 angeschlossen, an den eine Schwefelsäurezuleitung 222 mündet und aus dem das Rohacetoncyanhydrin durch die Ableitung 223 in die Acetoncyanhydrinaufarbeitung 30 geführt wird.

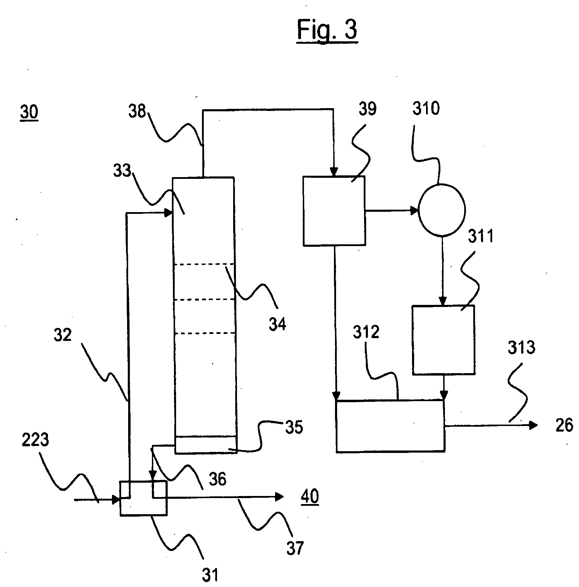

In Figur 3 mündet aus der Cyanhydrinherstellung 20 kommend die Ableitung 223 in einen Wärmetauscher 31, in dem der aus der Cyanherstellung 20 kommende Strom erwärmt wird. An den Wärmetauscher 31 schließt sich eine Brüdenzuleitung 32 an, die in dem oberen, vorzugsweise dem Kopfbereich einer Kolonne 33 mündet. Die Kolonne 33 weist eine Vielzahl von Packungen 34 auf, die meist als Böden ausgestaltet sind. Im unteren Bereich der Kolonne 33 befindet sich der Kolonnensumpf 35 aus dem eine Sumpfableitung 36 in den Wärmeaustauscher 31 führt und den über die Ableitung 233 in den Wärmeaustausch 31 geführten Ströme erwärmt. An den Wärmetauscher 31 schließt sich eine Reinproduktführung 37 an, auf die die Amidierung 40 stromab folgt. Im Kopfbereich der Kolonne 33 befindet sich eine Kopfableitung 38, die in einen Wärmetauscher 39 mündet, an den sich eine Vakuumpumpe 310 anschließt, die wiederum in einen Wärmetauscher 311 mündet. Sowohl der Wärmetauscher 39 als auch der Wärmetauscher 311 sind über Leitungen mit einem Kühlbehälter 312 verbunden an den sich eine Rückführung 313 anschließt, die mit dem Schlaufenreaktor 26 in der Acetoncyanhydrinherstellung 20 verbunden ist.

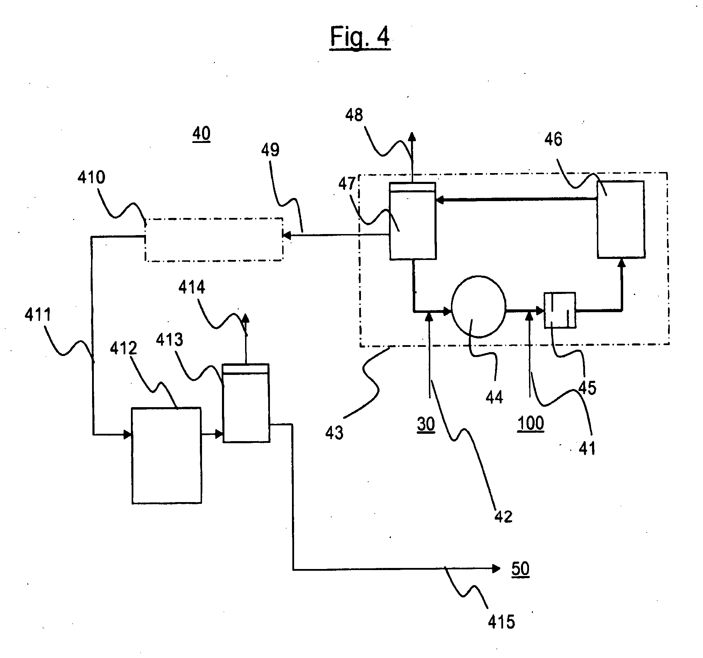

Die in Fig. 4 abgebildete Amidierung 40 weist zunächst eine Acetoncyanhydrinzuführung 41 und eine Schwefelsäurezuführung 42 auf, die in einen Schlaufenreaktor 43 münden. Die mit der Acetoncyanhydrinaufarbeitung 30 verbundene Acetoncyanhydrinzuführung 41 mündet in den Kreislauf des Schlaufenreaktors 43 nach einer Pumpe 44 vor einem Mischer 45. Vor dieser Pumpe 44 mündet die Schwefelsäurezuführung 42. Stromab des Mischers 45 folgt ein Wärmeaustauscher 46 der wiederum in einen Gasabscheider 47 mündet aus dem zum einen eine Gasableitung 48 und eine Zuleitung 49 zu einem weiteren Schlaufenreaktor 410 abgeht. Der weitere Schlaufenreaktor 410 oder ein Dritter ist vergleichbar wie der erste Schlaufenreaktor 43 aufgebaut. Von dem weiteren Schlaufenreaktor 410 geht eine Zuleitung 411 in einen Wärmeaustauscher 412, auf den ein Gasabscheider 413 folgt, von dem zu einem eine Gasableitung 414 und eine Amidleitung 415 abgeht, die zu der Veresterung/Verseifung 50 /MAS-Anlage 50a führt.

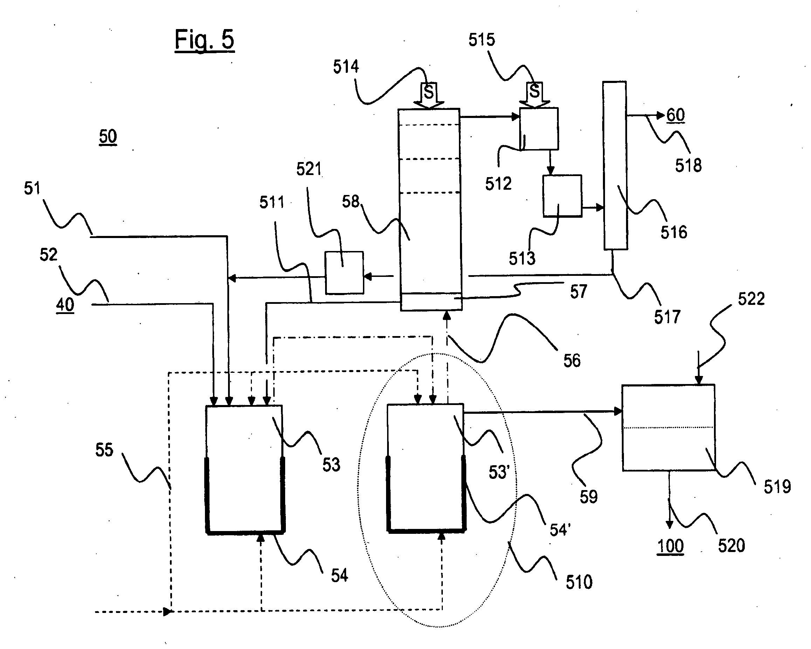

Die Figur 5 zeigt die Veresterung 50, in der eine Wasser und organische Lösungsmittel führende Lösungsmittelleitung 51 und eine mit der Amidierung 40 verbundene Amidleitung 52 in einen Kessel 53 münden, der durch eine Kesselheizung 54 beheizbar ist. In den Kessel 53 mündet weiterhin eine gestrichelt gezeichnete Alkoholleitung 55. Die Alkoholleitung 55 mündet sowohl in dem oberen als auch in dem unteren Bereich des Kessels 53. Über eine mit Strichpunkt gekennzeichnete Esterbrüdenleitung 56 ist der erste Kessel 53 mit einem weiteren Kessel 53' verbunden, der eine weitere Kesselheizung 54' aufweist. Auch dieser weitere Kessel 53' ist sowohl von unten als auch von oben mit der Alkoholleitung 55 verbunden. An den oberen Bereich des Kessels 53' schließt sich die Esterbrüdenleitung 56 an, die in einen Sumpf 57 einer Kolonne 58 mündet. Weiterhin befindet sich im oberen Bereich des Kessels 53' eine Leitung für verdünnte Schwefelsäure 59. Eine in der gepunkteten Ellipse eingerahmte Kesseleinheit 510 wird aus einem beheizbaren Kessel 53 und 54 mit Alkoholleitung 55 und Esterbrüdenleitung 56 gebildet. Es können ein, zwei oder mehr derartiger Kesseleinheiten kaskadenartig aufeinander folgen, wobei jede dieser Kesseleinheiten 510 über die Esterbrüdenleitung 56 mit dem Sumpf 57 der Kolonne 58 verbunden ist. Aus dem Sumpf 57 der Kolonne 58 führt weiterhin eine Schwersiederleitung 511 zum Kessel 53, um Wasser und organische Lösungsmittel wieder der Veresterung zuzuführen. Im Oberbereich, vorzugsweise dem Kopf, der Kolonne 58, schließt sich über eine geeignete Leitung ein erster Wärmetauscher 512 gefolgt von einem weiteren Phasentrenner 513 an. Sowohl am Kopf der Kolonne 58 als auch in dem ersten Wärmetauscher 512 können eine erste Stabilisatorzuführung 414 (Stabilisator mit "S" gekennzeichnet) sowie eine weitere Stabilisatorzuführung 515 vorgesehen sein, um einen eine unerwünschte Polymerisation verhindernden Inhibitor bzw. Stabilisator zuzuführen. An den weiteren Phasentrenner 513 schließt sich ein Wäscher 516 an, in dessen unteren Bereich eine Lösemittelleitung 517 abgeht, die über einen Wärmetauscher 521 in der Lösungsmittelleitung 51 mündet. Von dem oberen Bereich des Wäschers 516 geht eine Rohesterleitung ab, die in die Esteraufarbeitung 60 mündet. Die aus dem oberen Bereich des Kessels 53' bzw. des Kessels der letzten Kesseleinheit 510 abgehende Spendsäureleitung 59 mündet in einen Flotationsbehälter 519 zum Abtrennen der Feststoffe bzw. in der Spendsäure nicht löslichen Bestandteile. Aus dem Flotationsbehälter 519 geht eine Spentsäureableitung 520 in die Schwefelsäureanlage 100 sowie eine, die niedriegsiedenden Bestandteile führende, Niedersiederbrüdenleitung 522 zur weiteren Aufarbeitung und Rückführung in die Veresterung.

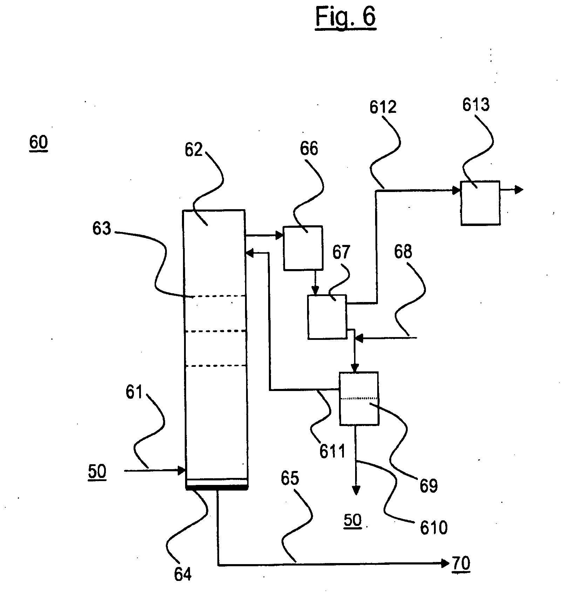

Die in Figur 6 gezeigt Esteraufarbeitung schließt sich über eine Rohesterleitung 61 an die Veresterung 50 an, wobei die Rohesterzuleitung 61 in den mittleren Bereich einer Vakuumdestillationskolonne 62 mündet. Diese Kolonne 62 weist Kolonneneinbauten 63 und eine im unteren Bereich der Kolonne 62 angeordnete Sumpfheizung 64 auf. Von dem unteren Bereich der Kolonne 62, der den Sumpf dieser Kolonne darstellt, geht eine Esterableitung 65 ab, die in die Esterfeinreinigung 70 mündet und den von Leichtsiedern befreiten Rohester somit der Feinreinigung zuführt. Im oberen Bereich der Kolonne 62, meist im Kopf, schließt sich über eine Ableitung ein erster Wärmetauscher 66 und ein weiterer oder mehrere Wärmetauscher 67 an, auf den ein Phasentrenner 69 folgt. In dem Phasentrenner 69 wird die aus dem Wärmetauscher 67 stammende Mischung in organische und wässrige Bestandteile aufgeteilt, wobei sich an den Phasentrenner 69 eine Rückführung 611 im oberen Bereich anschließt, die im oberen Bereich der Kolonne 62 einmündet. Im unteren Bereich des Abscheiders befindet sich eine Wasserableitung 610, die in die Veresterung 50 mündet, um das abgetrennte Wasser der Versterung wieder zuzuführen. An die Wärmetauscher 66 und 67 schließt sich über eine Unterdruckleitung 612 eine Unterdruckerzeuger 613 an.

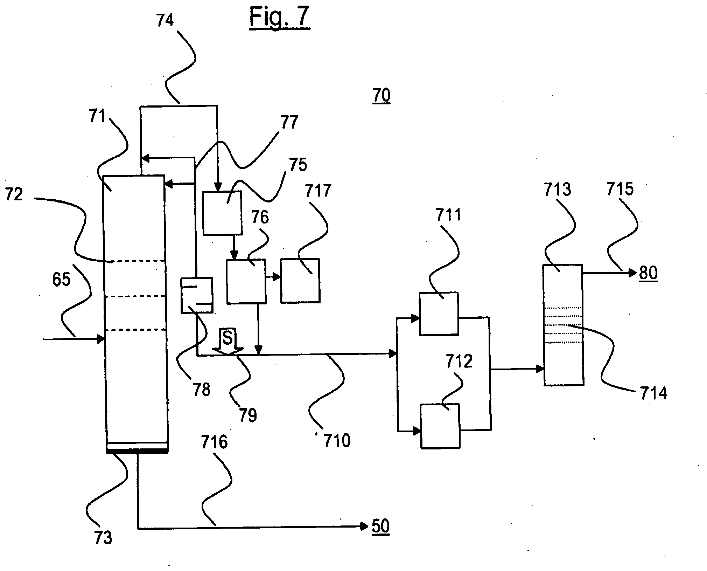

In Figur 7 mündet die aus der Esteraufarbeitung 60 stammende Esterableitung 65 in eine Destillationskolonne 71. Diese umfasst mehrere Kolonneneinbauten 71 sowie im unteren Bereich der Destillationskolonne 71 eine Kolonnensumpfheizung 73. Vom Kopfbereich der Destillationskolonne 71 geht eine Reinesterbrüdenleitung 74 in einen ersten Wärmetauscher 75, auf den ein (oder mehrere) weiterer Wärmetauscher 76 folgen, die mit einem Unterdruckerzeuger 717 verbunden sind. Der Ausgang des weiteren Wärmetauschers 76 weist eine Leitung auf, von der zum einen eine Esterrückführung 77 in den oberen Bereich der bzw. in den Kopf der Destillationskolonne 71 mündet. Die Esterrückführung 77 weist eine Stabilisatorzudosierung 79 auf, die in der Esterrückführung 77 vor einem Mischer 78 angeordnet ist. Zum anderen geht von der Leitung des weiteren Wärmetauschers 76 eine Reinesterableitung 710 ab. An diese schließt sich in Reihenschaltung ein zusätzlicher Wärmetauscher 711 und ein anderer Wärmetauscher 712 an. Auf diese folgt ein Molekularsiebbehälter 713, der Molekufarsiebpackungen 714 aufweist. Durch das Molekularsieb weiter gereinigt, wird der Reinstester durch die sich an den Molekularsiebbehälter anschließende Reinstesterableitung in die Weiterverarbeitungsanlage 80 überführt.

### Bezugszeichenliste

- 1: Anlagenverbund
- 2: Schwefelsäureleitung
- 3: weitere Schwefelsäureleitung
- 4: verbrauchte Schwefelsäureleitung - Ester
- 5: verbrauchte Schwefelsäureleitung - Säure
- 6: Methacrysäureleitung
- 20: Acetoncyanhydrinherstellung
- 30: Acetoncyanhydrinaufarbeitung
- 40: Amidierung
- 50: Veresterung
- 50a: Hydrolyse
- 60: Vorreinigung
- 70: Endreinigung
- 80: Weiterverarbeitungsanlage
- 90: Konfektionierung
- 100: Schwefelsäureanlage
- 21: Acetonbehälter
- 22: Blausäurebehälter
- 23: Waschturm
- 24: Kühlelemente
- 25: Abgasleitungen
- 26: Schlaufenreaktor
- 27: Acetonzuleitung
- 28: Blausäurezuleitung
- 29: Pumpe
- 210: Katalysatorzuleitung
- 211: Mischer
- 212: Wärmetauscher
- 213: Strömungswiderstand
- 214: Kühlleitungen
- 215: Wärmetauscher
- 216: Sammelbehälter
- 217: Düse
- 218: Kühlkreislauf
- 219: Wärmetauscher
- 220: Ableitung
- 221: Stabilisierbehälter
- 222: Schwefelsäurezuleitung
- 223: Ableitung
- 31: Wärmetauscher
- 32: Brüdenzuleitung
- 33: Kolonne
- 34: Packungen
- 35: Kolonnensumpf mit Wärmetauscher
- 36: Sumpfableitung
- 37: Reinproduktführung
- 38: Kopfableitung
- 39: Wärmetauscher
- 310: Vakuumpumpe
- 311: Wärmetauscher
- 312: Kühlbehälter
- 313: Rückführung
- 41: Acetoncyanhydrinzuführung
- 42: Schwefelsäurezuführung
- 43: Schlaufenreaktor
- 44: Pumpe
- 45: Mischer
- 46: Wärmetauscher
- 47: Gasabscheider
- 48: Gasableitung
- 49: Zuleitung
- 410: weiterer Schlaufenreaktor
- 411: Zuleitung
- 412: Wärmetauscher
- 413: Gasabscheider
- 414: Gasableitung
- 415: Amidleitung
- 51: Lösungsmittelleitung
- 52: Amidleitung
- 53: erster Kessel
- 54: erste Kesselheizung
- 53': weiterer Kessel
- 54': weitere Kesselheizung
- 55: Alkoholleitung
- 56: Esterbrüdenleitung
- 57: Kolonnensumpf
- 58: Kolonne
- 59: Spendsäureleitung
- 510: Kesseleinheit
- 511: Schwersiederleitung
- 512: Wärmetauscher
- 513: Phasentrenner
- 514: Stabilisatorzuführung
- 515: weitere Stabilisatorzuführung
- 516: Extraktionskolonne
- 517: Lösemittelleitung
- 518: Rohesterleitung
- 519: Flotationsbehälter
- 520: Spentsäureableitung
- 521: Wärmetauscher
- 522: Niedersiederbrüdenleitung
- 61: Rohesterzuleitung
- 62: Vakuumdestillationskolonne
- 63: Kolonneneinbauten
- 64: Sumpfheizung
- 65: Esterableitung
- 66: Wärmetauscher
- 67: Wärmetauscher
- 68: Wasserzufuhr
- 69: Phasentrenner
- 610: Wasserableitung
- 611: Rückführung
- 612: Unterdruckleitung
- 613: Unterdruckerzeuger
- 71: Destillationskolonne
- 72: Kolonneneinbauten
- 73: Kolonnensumpfheizung
- 74: Reinesterbrüdenleitung
- 75: erster Wärmetauscher
- 76: weitere Wärmetauscher
- 77: Esterrückführung
- 78: Mischer
- 79: Stabilisatorzudosierung
- 710: Reinesterableitung
- 711: zusätzlicher Wärmetauscher
- 712: andere Wärmetauscher
- 713: Molekularsiebbehälter
- 714: Molekularsiebpackungen
- 715: Reinstesterableitung
- 716: Schwersiederleitung

## Patentansprüche

1. Verfahren zur Herstellung von Methacrylsäurealkylestern als Reaktionsprodukt bei dem
a. in einem oder mehreren Reaktionsräumen ein Methacrylsäureamid, Wasser, Schwefelsäure und mindestens ein Alkanol enthaltendes Reaktionsgemisch einer Veresterungsreaktion unterzogen wird,
b. das rohe Reaktionsprodukt in mindestens einer Rektifikationskolonne einer Trennoperation unterzogen wird,
c. das einer Trennoperation unterworfene Reaktionsprodukt in einem oder mehreren Wärmetauschern kondensiert wird,
d. das Kondensat in mindestens einer Scheidevorrichtung in eine organische und eine wässrige Phase getrennt wird,
e. die organische Phase mit Wasser unter Erhalt einer gewaschenen organischen Phase und Waschwasser gewaschen wird und
f. die abgetrennte wässrige Phase zusammen mit dem Waschwasser wieder in mindestens einen Reaktionsraum zurückgeführt wird.

2. Verfahren nach Anspruch 1, **dadurch gekennzeichnet, dass** als Reaktionsraum ein Kessel oder eine Kaskade von 2, 3, 4 oder mehr Kesseln eingesetzt wird.

3. Verfahren nach einem der Ansprüche 1 oder 2, **dadurch gekennzeichnet, dass** dem Reaktionsraum oder dem ersten von zwei oder mehr Reaktionsräumen zumindest anteilig Wasser zugeführt wird, das Alkanol und Schwefelsäure enthält.

4. Verfahren nach einem der vorstehenden Ansprüche, **dadurch gekennzeichnet, dass** die Temperatur im Reaktionsraum auf etwa 90 bis etwa 120 °C eingestellt wird und dem Reaktionsgemisch aus einem oder mehreren Reaktionsräumen dampfförmiges Reaktionsprodukt entzogen wird, das über eine Rektifikationskolonne einer Trennoperation unterzogen, in einem oder mehreren Wärmetauschern kondensiert und als Kondensat anschließend mit Wasser gewaschen wird, wobei das so erhaltene Waschwasser vom Reaktionsprodukt durch Phasentrennung abgetrennt und wieder in mindestens einen Reaktionsraum zurückgeführt wird.

5. Verfahren nach einem der vorstehenden Ansprüche, **dadurch gekennzeichnet, dass** es die folgenden Schritte umfasst:
a. mindestens einem Reaktionsraum wird ein Gemisch enthaltend Wasser, Ammoniumsalze, Reaktionsprodukt, Feststoffe und Schwefelsäure (verbrauchte Säure) entzogen,
b. das Gemisch wird mindestens einem Flotationsbehälter zugeführt wird in dem es durch Flotation weitgehend von Feststoffen befreit wird,
c. der Flotationsbehälter wird derart mit Dampf beaufschlagt, dass das Reaktionsprodukt aus dem Gemisch in die Dampfphase überführt wird,
d. die mindestens Wasser und Reaktionsprodukt enthaltende Dampfphase wird in mindestens einem Wärmetauscher so gekühlt wird, dass ein Wasser und Reaktionsprodukt enthaltendes Nachkondensat entsteht und
e. das Kondensat wird in mindestens einen Reaktionsraum zurückgeführt.

6. Verfahren nach Anspruch 5, **dadurch gekennzeichnet, dass** die Innenseite mindestens eines Wärmetauschers mit Waschwasser aus der Wäsche des Kondensats aus der Rektifikationskolonne des Vorreinigungsschritts besprüht wird.

7. Verfahren nach Anspruch 6, **dadurch gekennzeichnet, dass** im Wärmetauscher ein Gemisch aus Nachkondensat und Waschwasser entsteht, das in mindestens einen Reaktionsraum zurückgeführt wird.

8. Verfahren nach einem der vorstehenden Ansprüche, **dadurch gekennzeichnet, dass** das Reaktionsprodukt mindestens einer Vorreinigung und mindestens einer Hauptreinigung unterzogen wird, wobei das Reaktionsprodukt bei der Vorreinigung destillativ überwiegend von niedrigsiedenden Verunreinigungen befreit wird, die einen niedrigeren Siedepunkt als der hergestellte Methacrylsäurealkylester aufweisen sowie von deren azeoptropen Gemischen mit Wasser und bei der Hauptreinigung überwiegend von höhersiedenden Verunreinigungen befreit wird, die einen höheren Siedepunkt als der hergestellte Methacrylsäurealkylester aufweisen.

9. Verfahren nach Anspruch 8, **dadurch gekennzeichnet, dass** die niedrigsiedenden Verunreinigungen einer Kondensation in einem oder mehreren Wärmetauschern unterzogen werden, das Kondensat einer Phasentrennung unterzogen wird und die wässrige Phase des Kondensats in mindestens einen Reaktionsraum zurückgeführt wird.

10. Verfahren nach einem der vorstehenden ,Ansprüche, **dadurch gekennzeichnet, dass** mindestens ein Reaktionsraum mit einem Gemisch aus Methacrylsäureamid und konzentrierter Schwefelsäure beschickt wird.

11. Verfahren nach einem der vorstehenden Ansprüche, **dadurch gekennzeichnet, dass** eine Kaskade von 2, 3 oder 4 Kesseln als Reaktionsräume eingesetzt werden.

12. Verfahren nach einem der vorstehenden Ansprüche, **dadurch gekennzeichnet, dass** das im Reaktionsgemisch vorliegenden Wasser zu mindestens 60 Gew.-% aus einem oder mehreren der nachstehenden Verfahrenselemente stammt:
a. Wäsche des rohen Reaktionsprodukts nach einer ersten Trennoperation mit Wasser und anschließendes Abtrennen der wässrigen Phase durch Phasentrennung,
b. Austreiben von Reaktionsprodukt aus der verbrauchten Säure mit Wasserdampf und anschließende Kondensation des so erhaltenen Gemischs aus Reaktionsprodukt und Wasserdampf
c. destillatives Abtrennen niedrigsiedender Verunreinigungen und deren azeotrope mit Wasser aus dem rohen Reaktionsprodukt, Kondensieren der abgetrennten Verunreinigungen, versetzen des Kondensats mit Wasser und abtrennen der wässrigen Phase durch Phasentrennung.

13. Verfahren nach einem der vorstehenden Ansprüche, **dadurch gekennzeichnet, dass** es ein oder mehrere der folgenden Verfahrenselemente umfasst:
a. Herstellung von Acetoncyanhydrin aus Aceton und Blausäure;
b. Reinigung des Acetoncyanhydrins in einer Rektifikationskolonne;
c. Umsetzung des Acetoncyanhydrins zu Methacrylsäureamid.

14. Vorrichtung zur Herstellung von Methacrylsäurealkylester mindestens umfassend
a. einen oder mehrere Reaktionsräume in denen ein Methacrylsäureamid, Wasser Schwefelsäure und ein Alkanol enthaltendes Reaktionsgemisch einer Veresterungsreaktion unterzogen werden kann,
b. mindestens eine Rektifikationskolonne, in der das Reaktionsprodukt einer Trennoperation unterzogen werden kann,
c. einen oder mehrere Wärmetauscher, in denen das einer Trennoperation unterworfene Reaktionsprodukt kondensiert werden kann,
d. mindestens eine Scheidevorrichtung, in der das Kondensat in eine organische und eine wässrige Phase getrennt werden kann,
e. mindestens eine Waschkolonne, in der die organische Phase mit Wasser gewaschen werden kann und,
f. mindestens eine fluidleitende Verbindung zwischen Scheidevorrichtung und mindestens einem Reaktionsraum, durch welche die abgetrennte wässrige Phase, gegebenenfalls zusammen mit dem Waschwasser, wieder in mindestens einen Reaktionsraum zurückgeführt werden kann.

15. Vorrichtung nach Anspruch 14, **dadurch gekennzeichnet, dass** sie weiterhin mindestens umfasst:
a. mindestens eine Destillationskolonne, in welcher das rohe Reaktionsprodukt von Verunreinigungen mit einem niedrigeren Siedepunkt als der Methacrylsäurealkylester sowie von deren azeotropen mit Wasser befreit werden kann;
b. mindestens einen Wärmetauscher, in dem die dampfförmigen abgetrennten Verunreinigungen sowie deren azeotrope mit Wasser unter Bildung eines Kondensats kondensiert werden können,
c. mindestens eine Zuleitung für Wasser, in dem das Kondensat mit Wasser versetzt wird;
d. mindestens eine Scheidevorrichtung, in der eine wässrige Phase des Kondensats von einer organischen Phase getrennt werden kann und
e. mindestens eine fluidleitende Verbindung zwischen der wässrigen Phase und mindestens einem Reaktionsraum.

16. Vorrichtung nach Anspruch 14 oder 15, **dadurch gekennzeichnet, dass** sie weiterhin mindestens umfasst:
f. Mindestens einen Behälter (Flotationsbehälter), in dem verbrauchte Säure aus der Veresterungsreaktion zumindest teilweise von Feststoffen befreit werden kann;
g. mindestens eine fluidleitende Verbindung zwischen Flotationsbehälter und einem Reaktionsraum;
h. mindestens einen Wärmetauscher zur Kondensation von aus dem Flotationsbehälter entweichenden dampfförmigen Stoffen,
i. mindestens eine fluidleitende Verbindung zwischen Scheidevorrichtung und Wärmetauscher, die eine Zufuhr der in der Scheidevorrichtung erhaltenen wässrigen Phase in das Innere des Wärmetauschers ermöglicht;
j. mindestens eine fluidleitende Verbindung zwischen Wärmetauscher und mindestens einem Reaktionsraum.

## Claims

1. Process for preparing alkyl methacrylates as the reaction product by
a. subjecting a reaction mixture comprising methacrylamide, water, sulphuric acid and at least one alkanol to an esterification reaction in one or more reaction chambers,
b. subjecting the crude reaction product to a separating operation in at least one rectification column,
c. condensing the reaction product which has been subjected to a separating operation in one or more heat exchangers,
d. separating the condensate into an organic phase and an aqueous phase in at least one separating apparatus,
e. washing the organic phase with water to obtain a washed organic phase and washing water, and
f. recycling the aqueous phase removed together with the washing water back into at least one reaction chamber.

2. Process according to Claim 1, **characterized in that** the reaction chamber used is one tank or a battery of 2, 3, 4 or more tanks.

3. Process according to one of Claims 1 or 2, **characterized in that** water which comprises alkanol and sulphuric acid is fed at least partly to the reaction chamber or to the first of two or more reaction chambers.

4. Process according to one of the preceding claims, **characterized in that** the temperature in the reaction chamber is adjusted to about 90 to about 120°C, and vaporous reaction product is withdrawn from the reaction mixture from one or more reaction chambers and is subjected to a separating operation by means of a rectification column, condensed in one or more heat exchangers and subsequently washed with water as the condensate, the washing water thus obtained being removed from the reaction product by phase separation and recycled back into at least one reaction chamber.

5. Process according to one of the preceding claims, **characterized in that** it comprises the following steps:
a. a mixture comprising water, ammonium salts, reaction product, solids and sulphuric acid (spent acid) is withdrawn from at least one reaction chamber,
b. the mixture is fed to at least one flotation vessel, in which it is largely freed of solids by flotation,
c. the flotation vessel is charged with steam to the effect that the reaction product is converted from the mixture into the vapour phase,
d. the vapour phase comprising at least water and reaction product is cooled in at least one heat exchanger so as to form a postcondensate comprising water and reaction product, and
e. the condensate is recycled into at least one reaction chamber.

6. Process according to Claim 5, **characterized in that** the inside of at least one heat exchanger is sprayed with washing water from the washing of the condensate from the rectification column of the prepurification step.

7. Process according to Claim 6, **characterized in that** a mixture of postcondensate and washing water forms in the heat exchanger and is recycled into at least one reaction chamber.

8. Process according to one of the preceding claims, **characterized in that** the reaction product is subjected to at least one prepurification and at least one main purification, the reaction product in the prepurification being freed by distillation predominantly of low-boiling impurities which have a lower boiling point than the alkyl methacrylate prepared, and also of azeotropic mixtures thereof with water, and being freed in the main purification predominantly of higher-boiling impurities which have a higher boiling point than the alkyl methacrylate prepared.

9. Process according to Claim 8, **characterized in that** the low-boiling impurities are subjected to a condensation in one or more heat exchangers, the condensate is subjected to a phase separation and the aqueous phase of the condensate is recycled into at least one reaction chamber.

10. Process according to one of the preceding claims, **characterized in that** at least one reaction chamber is charged with a mixture of methacrylamide and concentrated sulphuric acid.

11. Process according to one of the preceding claims, **characterized in that** a battery of 2, 3 or 4 tanks is used as reaction chambers.

12. Process according to one of the preceding claims, **characterized in that** the water present in the reaction mixture stems to an extent of at least 60% by weight from one or more of the following process elements:
a. washing of the crude reaction product after a first separating operation with water and subsequently removing the aqueous phase by phase separation,
b. driving reaction product out of the spent acid with steam and subsequently condensing the mixture of reaction product and steam thus obtained,
c. distillatively removing low-boiling impurities and their azeotropes with water from the crude reaction product, condensing the impurities removed, admixing the condensate with water and removing the aqueous phase by phase separation.

13. Process according to one of the preceding claims, **characterized in that** it comprises one or more of the following process elements:
a. preparing acetone cyanohydrin from acetone and hydrocyanic acid;
b. purifying the acetone cyanohydrin in a rectification column;
c. converting the acetone cyanohydrin to methacrylamide.

14. Apparatus for preparing alkyl methacrylate, at least comprising
a. one or more reaction chambers in which a reaction mixture comprising methacrylamide, water, sulphuric acid and an alkanol can be subjected to an esterification reaction,
b. at least one rectification column in which the reaction product can be subjected to a separating operation,
c. one or more heat exchangers in which the reaction product subjected to a separating operation can be condensed,
d. at least one separating apparatus in which the condensate can be separated into an organic phase and an aqueous phase,
e. at least one washing column in which the organic phase can be washed with water, and
f. at least one fluid-conducting connection between separating apparatus and at least one reaction chamber, through which the removed aqueous phase, if appropriate together with the washing water, can be recycled back into at least one reaction chamber.

15. Apparatus according to Claim 14, **characterized in that** it further at least comprises:
a. at least one distillation column in which the crude reaction product can be freed of impurities having a boiling point lower than that of the alkyl methacrylate and of azeotropes thereof with water;
b. at least one heat exchanger in which the vaporous impurities removed and their azeotropes with water can be condensed to form a condensate;
c. at least one feedline for water, in which the condensate is admixed with water;
d. at least one separating apparatus in which an aqueous phase of the condensate can be separated from an organic phase and
e. at least one fluid-conducting connection between the aqueous phase and at least one reaction chamber.

16. Apparatus according to Claim 14 or 15, **characterized in that** it further at least comprises:
f. at least one vessel (flotation vessel) in which spent acid from the esterification reaction can be freed at least partly of solids;
g. at least one fluid-conducting connection between flotation vessel and a reaction chamber;
h. at least one heat exchanger for condensing vaporous substances escaping from the flotation vessel;
i. at least one fluid-conducting connection between separating apparatus and heat exchanger, which enables feeding of the aqueous phase obtained in the separating apparatus into the interior of the heat exchanger;
j. at least one fluid-conducting connection between heat exchanger and at least one reaction chamber.

## Revendications

1. Procédé pour la préparation d'esters alkyliques de l'acide méthacrylique comme produit de réaction, dans lequel
a. un mélange réactionnel contenant de l'amide de l'acide méthacrylique, de l'eau, de l'acide sulfurique et au moins un alcanol est soumis dans une ou plusieurs chambres de réaction à une réaction d'estérification,
b. le produit de réaction brut est soumis dans au moins une colonne de rectification à une opération de séparation,
c. le produit de réaction soumis à une opération de séparation est condensé dans un ou plusieurs échangeurs thermiques,
d. le condensat est séparé dans au moins un dispositif de séparation en une phase organique et une phase aqueuse,
e. la phase organique est lavée avec de l'eau avec obtention d'une phase organique lavée et de l'eau de lavage et,
f. la phase aqueuse séparée est à nouveau recyclée, ensemble avec l'eau de lavage, dans au moins une chambre de réaction.

2. Procédé selon la revendication 1, **caractérisé en ce qu'**on utilise, comme chambre de réaction, une cuve ou une cascade de 2, 3, 4 cuves ou plus.

3. Procédé selon l'une quelconque des revendications 1 ou 2, **caractérisé en ce que** la chambre de réaction ou la première de deux chambres de réaction ou plus est au moins partiellement alimentée en eau, qui contient l'alcanol et l'acide sulfurique.

4. Procédé selon l'une quelconque des revendications précédentes, **caractérisé en ce que** la température dans la chambre de réaction est réglée à environ 90 jusqu'environ 120 °C et un produit de réaction sous forme de vapeur est soutiré du mélange réactionnel dans une ou plusieurs chambres de réaction, qui est soumis sur une colonne de rectification à une opération de séparation, condensé dans un ou plusieurs échangeurs thermiques et ensuite lavé, en tant que condensat, avec de l'eau, l'eau de lavage ainsi obtenue étant séparée du produit de réaction par séparation de phases et à nouveau recyclée dans au moins une chambre de réaction.

5. Procédé selon l'une quelconque des revendications précédentes, **caractérisé en ce qu'**il comprend les étapes suivantes :
a. un mélange contenant de l'eau, des sels d'ammonium, le produit de réaction, des solides et de l'acide sulfurique (acide consommé) est soutiré d'au moins une chambre de réaction,
b. le mélange est introduit dans au moins un récipient de flottation, dans lequel il est libéré par flottation, dans une large mesure, des solides,
c. le récipient de flottation est alimenté en vapeur de manière telle que le produit de réaction est transféré du mélange dans la phase vapeur,
d. la phase vapeur contenant au moins l'eau et le produit de réaction est refroidie dans au moins un échangeur thermique de manière telle qu'il se forme un post-condensat contenant de l'eau et du produit de réaction et
e. le condensat est recyclé dans au moins une chambre de réaction.

6. Procédé selon la revendication 5, **caractérisé en ce que** la face interne d'au moins un échangeur thermique est aspergée d'eau de lavage provenant du lavage du condensat de la colonne de rectification de l'étape de prépurification.

7. Procédé selon la revendication 6, **caractérisé en ce qu'**il se forme dans l'échangeur thermique un mélange de post-condensat et d'eau de lavage, qui est recyclé dans au moins une chambre de réaction.

8. Procédé selon l'une quelconque des revendications précédentes, **caractérisé en ce que** le produit de réaction est soumis à au moins une prépurification et à au moins une purification principale, le produit de réaction étant libéré lors de la prépurification par distillation principalement des impuretés à bas point d'ébullition, qui présentent un point d'ébullition inférieur à celui de l'ester alkylique de l'acide méthacrylique préparé ainsi que de leurs mélanges azéotropes avec l'eau et lors de la purification principale principalement des impuretés à point d'ébullition plus élevé, qui présentent un point d'ébullition supérieur à celui de l'ester alkylique de l'acide méthacrylique préparé.

9. Procédé selon la revendication 8, **caractérisé en ce que** les impuretés à bas point d'ébullition sont soumises à une condensation dans un ou plusieurs échangeurs thermiques, le condensat est soumis à une séparation des phases et la phase aqueuse du condensat est recyclée dans au moins une chambre de réaction.

10. Procédé selon l'une quelconque des revendications précédentes, **caractérisé en ce qu'**au moins une chambre de réaction reçoit un mélange d'amide de l'acide méthacrylique et d'acide sulfurique concentré.

11. Procédé selon l'une quelconque des revendications précédentes, **caractérisé en ce qu'**on utilise une cascade de 2, 3 ou 4 cuves comme chambres de réaction.

12. Procédé selon l'une quelconque des revendications précédentes, **caractérisé en ce que** l'eau présente dans le mélange réactionnel provient à raison d'au moins 60 % en poids d'un ou de plusieurs des éléments de procédé suivantes :
a. le lavage du produit de réaction brut après une première opération de séparation avec de l'eau et séparation consécutive de la phase aqueuse par séparation de phases,
b. l'expulsion du produit de réaction de l'acide consommé par de la vapeur d'eau et condensation consécutive du mélange ainsi obtenu de produit de réaction et de vapeur d'eau
c. la séparation par distillation d'impuretés de bas point d'ébullition et de leurs azéotropes avec l'eau du produit de réaction brut, condensation des impuretés séparées, addition d'eau au condensat et séparation de la phase aqueuse par séparation des phases.

13. Procédé selon l'une quelconque des revendications précédentes, **caractérisé en ce qu'**il comprend un ou plusieurs des éléments de procédé suivantes :
a. préparation d'acétone-cyanhydrine à partir d'acétone et d'acide cyanhydrique ;
b. purification de l'acétone-cyanhydrine dans une colonne de rectification ;
c. transformation de l'acétone-cyanhydrine en amide de l'acide méthacrylique.

14. Dispositif pour la préparation d'esters alkyliques de l'acide méthacrylique, comprenant au moins
a. une ou plusieurs chambres de réaction dans laquelle/lesquelles un mélange réactionnel contenant de l'amide de l'acide méthacrylique, de l'eau, de l'acide sulfurique et un alcanol peut être soumis à une réaction d'estérification,
b. au moins une colonne de rectification dans laquelle le produit de réaction peut être soumis à une opération de séparation,
c. un ou plusieurs échangeurs thermiques, dans lequel/lesquels le produit de réaction soumis à une opération de séparation peut être condensé,
d. au moins un dispositif de séparation, dans lequel le condensat peut être séparé en une phase organique et une phase aqueuse,
e. au moins une colonne de lavage, dans laquelle la phase organique peut être lavée à l'eau et
f. au moins une liaison fluidique entre le dispositif de séparation et au moins une chambre de réaction, par laquelle la phase aqueuse séparée peut être recyclée, le cas échéant ensemble avec l'eau de lavage, à nouveau dans au moins une chambre de réaction.

15. Dispositif selon la revendication 14, **caractérisé en ce qu'**il comprend en outre au moins :
a. au moins une colonne de distillation dans laquelle le produit de réaction brut peut être libéré d'impuretés présentant un point d'ébullition plus bas que celui de l'ester alkylique de l'acide méthacrylique ainsi que de leurs azéotropes avec l'eau ;
b. au moins un échangeur thermique dans lequel les impuretés séparées sous forme de vapeur ainsi que leurs azéotropes avec l'eau peuvent être condensés avec formation d'un condensat,
c. au moins une conduite d'alimentation pour l'eau, dans laquelle le condensat est mélangé avec de l'eau ;
d. au moins un dispositif de séparation, dans lequel une phase aqueuse du condensat peut être séparée d'une phase organique et
e. au moins une liaison fluidique entre la phase aqueuse et au moins une chambre de réaction.

16. Dispositif selon la revendication 14 ou 15, **caractérisé en ce qu'**il comprend en outre au moins :
f. au moins un récipient (récipient de flottation) dans lequel l'acide consommé de la réaction d'estérification peut être au moins partiellement libéré des solides ;
g. au moins une liaison fluidique entre le récipient de flottation et une chambre de réaction ;
h. au moins un échangeur thermique pour la condensation de substances sous forme de vapeur s'échappant du récipient de flottation,
i. au moins une liaison fluidique entre le dispositif de séparation et l'échangeur thermique, qui permet une alimentation de la phase aqueuse obtenue dans le dispositif de séparation à l'intérieur de l'échangeur thermique ;
j. au moins une liaison fluidique entre l'échangeur thermique et au moins une chambre de réaction.
